(19) Europäisches Patentamt
European Patent Office
Office européen des brevets

(11) Veröffentlichungsnummer : **0 555 183 A1**

(12) # EUROPÄISCHE PATENTANMELDUNG

(21) Anmeldenummer : **93810059.1**

(22) Anmeldetag : **29.01.93**

(51) Int. Cl.$^5$ : **C07D 401/12,** A01N 43/54, C07D 403/12

(30) Priorität : **07.02.92 CH 376/92**

(43) Veröffentlichungstag der Anmeldung : **11.08.93 Patentblatt 93/32**

(84) Benannte Vertragsstaaten : **AT BE CH DE DK ES FR GB GR IE IT LI NL PT SE**

(71) Anmelder : **CIBA-GEIGY AG**
**Klybeckstrasse 141**
**CH-4002 Basel (CH)**

(72) Erfinder : **Walter, Harald, Dr.**
**Rufacherstrasse 20**
**CH-4455 Basel (CH)**

(54) **Schädlingsbekämpfungsmittel.**

(57) Verbindungen der Formel

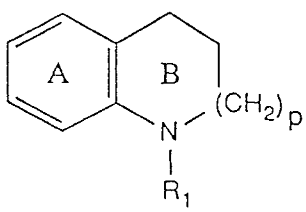

(I),

worin eines der (p+6) substituierbaren Ring-Kohlenstoffatome der beiden Ringe A und B durch eine Gruppe der Formel Ia

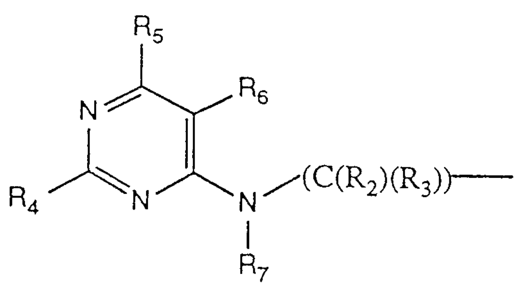

(Ia)

substituiert ist und worin die Variablen $R_1$ und p sowie die Ringe A und B die in Anspruch 1 angegebenen Bedeutungen haben, und gegebenenfalls Tautomere davon, jeweils in freier Form oder in Salzform, können als agrochemische Wirkstoffe verwendet werden und sind in an sich bekannter Weise herstellbar.

EP 0 555 183 A1

Die Erfindung betrifft Verbindungen der Formel

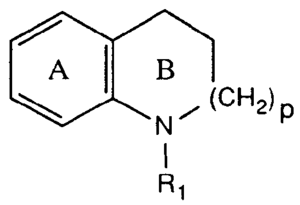

(I),

worin eines der (p + 6) substituierbaren Ring-Kohlenstoffatome der beiden Ringe A und B durch eine Gruppe der Formel Ia

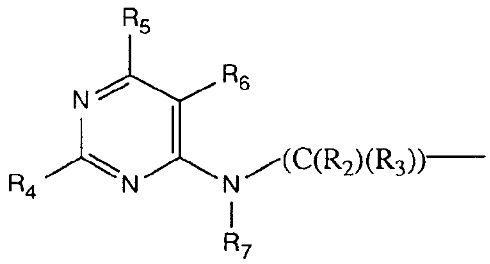

(Ia)

substituiert ist und die anderen (p + 5) substituierbaren Ring-Kohlenstoffatome der beid n Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_8$-Alkyl, Halogen-$C_1$-$C_8$-alkyl, $C_1$-$C_4$-Alkoxy-$C_1$-$C_4$-alkyl, $C_3$-$C_8$-Cycloalkyl, $C_1$-$C_8$-Alkoxy, Halogen-$C_1$-$C_8$-alkoxy, $C_3$-$C_8$-Cycloalkoxy, $C_1$-$C_8$-Alkylthio, Halogen-$C_1$-$C_8$-alkylthio, Cyano, Nitro, Phenyl, Phenoxy und Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_4$-Alkyl und $C_1$-$C_4$-Alkoxy, substituiert sind, mit der Massgabe, dass höchstens einer der gegebenenfalls vorhandenen Substituenten an den erwähnten (p + 5) substituierbaren Ring-Kohlenstoffatomen der beiden Ringe A und B gegebenenfalls wie vorstehend erwähnt substituiertes Phenyl, gegebenenfalls wie vorstehend erwähnt substituiertes Phenoxy oder gegebenenfalls wie vorstehend erwähnt substituiertes Phenylthio ist, besteht, ausgewählt sind;
wobei bedeuten:
$R_1$ Wasserstoff, $C_1$-$C_8$-Alkyl, $C_3$-$C_8$-Cycloalkyl, Benzyl, $C_1$-$C_8$-Alkanoyl, das unsubstituiert oder durch Halogen oder $C_1$-$C_4$-Alkoxy substituiert ist, Benzoyl, dessen Phenylgruppe unsubstituiert oder durch einen oder zwei Substituenten, ausgewählt aus der Gruppe bestehend aus Halogen, $C_1$-$C_4$-Alkyl, Nitro und Cyano, substituiert ist;
$C_1$-$C_8$-Alkansulfonyl, Halogen-$C_1$-$C_8$-alkansulfonyl, Cyano-$C_1$-$C_8$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_4$-Alkyl, Nitro und Cyano, substituiert ist;
$R_2$, $R_3$, unabhängig voneinander, Wasserstoff, $C_1$-$C_8$-Alkyl, Halogen-$C_1$-$C_8$-alkyl, $C_1$-$C_4$-Alkoxy-$C_1$-$C_4$-alkyl oder $C_3$-$C_8$-Cycloalkyl;
$R_4$ Wasserstoff, $C_1$-$C_8$-Alkyl, $C_1$-$C_8$-Alkoxy oder $C_1$-$C_8$-Alkylthio;
$R_5$ Wasserstoff, $C_1$-$C_8$-Alkyl, Halogen-$C_1$-$C_8$-alkyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkansulfinyl-$C_1$-$C_4$-alkyl, $C_1$-$C_4$-Alkansulfonyl-$C_1$-$C_4$-alkyl, $C_2$-$C_8$-Alkenyl, Halogen-$C_2$-$C_8$-alkenyl, $C_2$-$C_8$-Alkinyl, $C_3$-$C_8$-Cycloalkyl, $C_1$-$C_8$-Alkylthio oder Halogen;
$R_5$ Wasserstoff, Hydroxy, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, $C_1$-$C_8$-Alkylthio, $C_1$-$C_8$-Alkansulfinyl, $C_1$-$C_8$-Alkansulfonyl, Halogen, Nitro, Cyano, Amino, eine Gruppe der Formel $N(H)R_8$ (Ib), eine Gruppe der Formel $N(R_8)R_9$ (Ic) oder eine Gruppe der Formel $N{=}C(R_9)R_{10}$ (Id);
$R_7$ Wasserstoff, $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_4$-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei

Substituenten, ausgewählt aus der Gruppe bestehend aus Halogen, $C_1$-$C_4$-Alkyl, Nitro und Cyano, substituiert sind;

$R_8$ $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_4$-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind;

$R_9$ $C_1$-$C_8$-Alkyl;

$R_{10}$ Wasserstoff oder $C_1$-$C_8$-Alkyl;

p 1 oder 2;

und gegebenenfalls Tautomere davon, jeweils in freier Form oder in Salzform, ein Verfahren zur Herstellung und die Verwendung dieser Verbindungen und Tautomere, Schädlingsbekämpfungsmittel, deren Wirkstoff aus diesen Verbindungen und Tautomeren, jeweils in freier Form oder in agrochemisch verwendbarer Salzform, ausgewählt ist, ein Verfahren zur Herstellung und die Verwendung dieser Mittel und Zwischenprodukte für die Herstellung der Verbindungen der Formel I und ihrer Tautomeren.

Die Verbindungen I können teilweise als Tautomere vorliegen. Bedeutet z. B. der Rest $R_8$ in der Gruppe Ia Hydroxy, können entsprechende Verbindungen im Gleichgewicht mit den tautomeren Oxo-Derivaten, d. h. den entsprechenden Pyrimid-5-onen, stehen. Demgemäss sind unter den Verbindungen I nachstehend gegebenenfalls auch entsprechende Tautomere zu verstehen, auch wenn letztere nicht in jedem Fall speziell erwähnt werden.

Die Verbindungen I und gegebenenfalls ihre Tautomeren können als Salze vorliegen. Da die Verbindungen I mindestens ein basisches Zentrum aufweisen, können sie z. B. Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$-$C_4$-Alkancarbonsäuren, z.B. Essigsäure, wie gegebenenfalls ungesättigten Dicarbonsäuren, z.B. Oxal-, Malon-, Malein-, Fumar- oder Phthalsäure, wie Hydroxycarbonsäuren, z.B. Ascorbin-, Milch-, Apfel-, Wein- oder Zitronensäure, oder wie Benzoesäure, oder mit organischen Sulfonsäuren, wie gegebenenfalls, z.B. durch Halogen, substituierten $C_1$-$C_4$-Alkan- oder Aryl-sulfonsäuren, z. B. Methan- oder p-Toluolsulfonsäure, gebildet. Ferner können Verbindungen I mit mindestens einer aciden Gruppe Salze mit Basen bilden. Geeignete Salze mit Basen sind beispielsweise Metallsalze, wie Alkali- oder Erdalkalimetallsalze, z.B. Natrium-, Kalium-oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propyl-amin, oder einem Mono-, Di- oder Trihydroxyniederalkylamin, z.B. Mono-, Di- oder Triethanolamin. Weiterhin können gegebenenfalls entsprechende innere Salze gebildet werden. Bevorzugt sind im Rahmen der Erfindung agrochemisch vorteilhafte Salze; umfasst sind aber auch für agrochemische Verwendungen mit Nachteilen behaftete, z. B. bienen- oder fisch-toxische, Salze, die beispielsweise für die Isolierung bzw. Reinigung von freien Verbindungen I oder deren agrochemisch verwendbaren Salzen eingesetzt werden. Infolge der engen Beziehung zwischen den Verbindungen I in freier Form und in Form ihrer Salze sind vorstehend und nachfolgend unter den freien Verbindungen I bzw. ihren Salzen sinn- und zweckgemäss gegebenenfalls auch die entsprechenden Salze bzw. die freien Verbindungen I zu verstehen. Entsprechendes gilt für Tautomere von Verbindungen I und deren Salze.

Die vor- und nachstehend verwendeten Allgemeinbegriffe haben, sofern nicht abweichend definiert, die nachfolgend aufgeführten Bedeutungen.

Halogen - als Gruppe per se sowie als Strukturelement von anderen Gruppen und Verbindungen, wie von Halogenalkyl, Halogenalkenyl, Halogenalkoxy, Halogenalkylthio und Halogenalkansulfonyl, - ist Fluor, Chlor, Brom oder Iod, insbesondere Fluor, Chlor oder Brom.

Kohlenstoffhaltige Gruppen und Verbindungen enthalten, sofern nicht abweichend definiert, jeweils 1 bis und mit 8, vorzugsweise 1 bis und mit 4, insbesondere 1 oder 2, Kohlenstoffatome.

Alkyl - als Gruppe per se sowie als Strukturelement von anderen Gruppen und Verbindungen, wie von Halogenalkyl, Alkoxy, Halogenalkoxy, Alkoxyalkyl, Alkylthio, Cyanoalkylthio, Halogenalkylthio, Alkylthioalkyl, Alkansulfinyl, Alkansulfinylalkyl, Alkansulfonyl, Cyanoalkansulfonyl, Halogenalkansulfonyl und Alkansulfonylalkyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl, Hexyl, Heptyl oder Octyl, oder verzweigt, z. B. Isopropyl, Isobutyl, sek.-Butyl, tert.-Butyl, Isopentyl, Neopentyl oder Isooctyl.

Alkenyl - als Gruppe per se sowie als Strukturelement von anderen Gruppen und Verbindungen, wie von Halogenalkenyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig, z. B.

Ethenyl, Propen-1-yl, But-1-en-1-yl, Pent-2-en-1-yl oder Oct-3-en-1-yl, oder verzweigt, z. B. Propen-2-yl, But-1-en-2-yl oder Oct-2-en-4-yl.

Alkinyl ist, jeweils unter gebührender Berücksichtigung der von Fall zu Fall umfassten Anzahl Kohlenstoffatome, entweder geradkettig, z. B. Ethinyl, Propin-1-yl, But-1-in-1-yl, Pent-2-in-1-yl oder Oct-3-in-1-yl, oder verzweigt, z. B. Propin-2-yl, But-1-in-2-yl oder Oct-2-in-4-yl.

Alkanoyl ist, jeweils unter gebührender Berücksichtigung der von Fall zu Fall umfassten Anzahl Kohlenstoffatome, entweder geradkettig oder verzweigt, z. B. Formyl, Acetyl, Propionyl, Butyryl, Pivaloyl oder Octanoyl.

Cycloalkyl ist, jeweils unter gebührender Berücksichtigung der von Fall zu Fall umfassten Anzahl Kohlenstoffatome, Cyclopropyl, Cyclobutyl, Cyclopentyl, Cyclohexyl, Cycloheptyl oder Cyclooctyl.

Cycloalkoxy ist, jeweils unter gebührender Berücksichtigung der von Fall zu fall umfassten Anzahl Kohlenstoffatome, Cyclopropoxy, Cyclobutoxy, Cyclopentyloxy, Cyclohexyloxy, Cycloheptyloxy oder Cyclooctyloxy.

In Halogen-substituierten kohlenstoffhaltigen Gruppen und Verbindungen, wie Halogenalkyl, Halogenalkenyl, Halogenalkoxy, Halogenalkylthio und Halogenalkansulfonyl, ist mindestens eines der in der zugrundeliegenden nicht-halogenierten Grundstruktur vorhandenen Wasserstoffatome durch ein Halogenatom ersetzt; es können jedoch auch zwei oder mehr als zwei, z. B. - im Falle einer Perhalogenierung - alle, der in der zugrundeliegenden nicht-halogenierten Grundstruktur vorhandenen Wasserstoffatome unabhängig voneinander durch Halogenatome ersetzt sein. Beispielhaft erwähnt seien $-CH_2F$, $-CHF_2$, $-CF_3$, $-CH_2Cl$, $-CHCl_2$, $-CCl_3$, $-CCl_2CCl_3$, $-CH_2Br$, $-CH_2CH_2Br$, $-CHBrCl$, $-CCl=CCl_2$, $-OCH_2F$, $-SCHCl_2$ und $-SO_2CF_3$.

In Alkoxy-, Alkylthio-, Alkansulfinyl-, Alkansulfonyl- bzw. Cyano-substituierten kohlenstoffhaltigen Gruppen und Verbindungen, wie Alkoxyalkyl, Alkylthioalkyl, Alkansulfinylalkyl, Alkansulfonylalkyl, Cyanoalkylthio und Cyanoalkansulfonyl, ist eines der in der zugrundeliegenden unsubstituierten Grundstruktur vorhandenen Wasserstoffatome durch Alkoxy, Alkylthio, Alkansulfinyl, Alkansulfonyl bzw. Cyano ersetzt.

Bevorzugt sind im Rahmen der Erfindung

(1) Verbindungen der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_3$-$C_6$-Cycloalkyl, $C_1$-$C_4$-Alkoxy, Halogen-$C_1$-$C_4$-alkoxy mit 1, 2 oder 3 Halogenatomen, $C_3$-$C_7$-Cycloalkoxy, $C_1$-$C_4$-Alkylthio, Halogen-$C_1$-$C_4$-alkylthio mit 1, 2 oder 3 Halogenatomen, Cyano, Nitro, Phenyl, Phenoxy und Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_2$-Alkyl und $C_1$-$C_2$-Alkoxy, substituiert sind, mit der Massgabe, dass höchstens einer der gegebenenfalls vorhandenen Substituenten an den erwähnten (p + 5) substituierbaren Ring-Kohlenstoffatomen der beiden Ringe A und B gegebenenfalls wie vorstehend erwähnt substituiertes Phenyl, gegebenenfalls wie vorstehend erwähnt substituiertes Phenoxy oder gegebenenfalls wie vorstehend erwähnt substituiertes Phenylthio ist, besteht, ausgewählt sind;

wobei bedeuten:

$R_1$ Wasserstoff, $C_1$-$C_4$-Alkyl, $C_3$-$C_7$-Cycloalkyl, Benzyl, $C_1$-$C_4$-Alkanoyl, das unsubstituiert oder durch Halogen oder $C_1$-$C_2$-Alkoxy substituiert ist, Benzoyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;

$C_1$-$C_4$-Alkansulfonyl, Halogen-$C_1$-$C_4$-alkansulfonyl mit 1, 2 oder 3 Halogenatomen, Cyano-$C_1$-$C_4$-alkan sulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;

$R_2$, $R_3$, unabhängig voneinander, Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl oder $C_3$-$C_7$-Cycloalkyl;

$R_4$ Wasserstoff, $C_1$-$C_4$-Alkyl, $C_1$-$C_4$-Alkoxy oder $C_1$-$C_4$-Alkylthio;

$R_5$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$-$C_4$-alkyl, $C_1$-$C_2$-Alkansulfonyl-$C_1$-$C_4$-alkyl, $C_2$-$C_4$-Alkenyl, Halogen-$C_2$-$C_4$-alkenyl mit 1, 2 oder 3 Halogenatomen, $C_2$-$C_4$-Alkinyl, $C_3$-$C_7$-Cycloalkyl, $C_1$-$C_4$-Alkylthio oder Halogen;

$R_6$ Wasserstoff, Hydroxy, $C_1$-$C_4$-Alkyl, Halogen-$C_1$-$C_4$-alkyl mit 1, 2 oder 3 Halogenatomen, $C_1$-$C_2$-Alkoxy-

$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro, Cyano, Amino, eine Gruppe der Formel $N(H)R_8$ (Ib), eine Gruppe der Formel $N(R_8)R_9$ (Ic) oder eine Gruppe der Formel $N{=}C(R_9)R_{10}$ (Id);

$R_7$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;

$R_8$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-C4-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;

$R_9$ $C_1$-$C_4$-Alkyl;

$R_{10}$ Wasserstoff oder $C_1$-$C_4$-Alkyl;

p 1 oder 2;

oder gegebenenfalls ein Tautomeres davon, jeweils in freier Form oder in Salzform.

(2) Verbindungen der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass, wenn p 1 ist, die Gruppe Ia in 2-, 3- 6- oder 7-Position des in der Formel I dargestellten, aus den beiden Ringen A und B gebildeten, bicyclischen Ringsystems gebunden ist, mit der weiteren Massgabe, dass, wenn p 2 ist, die Gruppe Ia in 2-, 3-, 7- oder 8-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_3$-$C_6$-Cycloalkyl, $C_1$-$C_4$-Alkoxy, Halogen-$C_1$-$C_4$-alkoxy mit 1, 2 oder 3 Halogenatomen, $C_3$-$C_7$-Cycloalkoxy, $C_1$-$C_4$-Alkylthio, Halogen-$C_1$-$C_4$-alkylthio mit 1, 2 oder 3 Halogenatomen, Cyano, Nitro, Phenyl, Phenoxy und Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_2$-Alkyl und $C_1$-$C_2$-Alkoxy, substituiert sind, mit der Massgabe, dass höchstens einer der gegebenenfalls vorhandenen Substituenten an den erwähnten (p + 5) substituierbaren Ring-Kohlenstoffatomen der beiden Ringe A und B gegebenenfalls wie vorstehend erwähnt substituiertes Phenyl, gegebenenfalls wie vorstehend erwähnt substituiertes Phenoxy oder gegebenenfalls wie vorstehend erwähnt substituiertes Phenylthio ist, besteht, ausgewählt sind;

wobei bedeuten:

$R_1$ Wasserstoff, $C_1$-$C_4$-Alkyl, $C_3$-$C_7$-Cycloalkyl, Benzyl, $C_1$-$C_4$-Alkanoyl, das unsubstituiert oder durch Halogen oder $C_1$-$C_2$-Alkoxy substituiert ist, Benzoyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;

$C_1$-$C_4$-Alkansulfonyl, Halogen-$C_1$-$C_4$-alkansulfonyl mit 1, 2 oder 3 Halogenatomen, Cyano-$C_1$-$C_4$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;

$R_2$, $R_3$, unabhängig voneinander, Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl oder $C_3$-$C_7$-Cycloalkyl;

$R_4$ Wasserstoff, $C_1$-$C_4$-Alkyl, $C_1$-$C_4$-Alkoxy oder $C_1$-$C_4$-Alkylthio;

$R_5$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$-$C_4$-alkyl, $C_1$-$C_2$-Alkansulfonyl-$C_1$-$C_4$-alkyl, $C_2$-$C_4$-Alkenyl, Halogen-$C_2$-$C_4$-alkenyl mit 1, 2 oder 3 Halogenatomen, $C_2$-$C_4$-Alkinyl; $C_3$-$C_7$-Cycloalkyl, $C_1$-$C_4$-Alkylthio oder Halogen;

$R_6$ Wasserstoff, Hydroxy, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro,

Cyano, Amino, eine Gruppe der Formel $N(H)R_8$ (Ib), eine Gruppe der Formel $N(R_8)R_9$ (Ic) oder eine Gruppe der Formel $N{=}C(R_9)R_{10}$ (Id);

$R_7$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;

$R_8$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;

$R_9$ $C_1$-$C_4$-Alkyl;

$R_{10}$ Wasserstoff oder $C_1$-$C_4$-Alkyl;

p 1 oder 2;

oder gegebenenfalls ein Tautomeres davon, jeweils in freier Form oder in Salzform.

(3) Verbindungen der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass, wenn p 1 ist, die Gruppe Ia in 2-, 3- 6- oder 7-Position des in der Formel I dargestellten, aus den beiden Ringen A und B gebildeten, bicyclischen Ringsystems gebunden ist, mit der weiteren Massgabe, dass, wenn p 2 ist, die Gruppe Ia in 2-, 3-, 7- oder 8-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl und $C_1$-$C_4$-Alkoxy besteht, ausgewählt sind;

wobei bedeuten:

$R_1$ Wasserstoff, $C_1$-$C_4$-Alkyl oder $C_1$-$C_4$-Alkanoyl;

$R_2$, $R_3$, unabhängig voneinander, Wasserstoff oder $C_1$-$C_4$-Alkyl;

$R_4$ Wasserstoff oder $C_1$-$C_4$-Alkyl;

$R_5$ $C_1$-$C_4$-Alkyl, Halogen-$C_1$-$C_4$-alkyl mit 1, 2 oder 3 Halogenatomen, $C_2$-$C_4$-Alkinyl, $C_3$-$C_4$-Cycloalkyl oder $C_1$-$C_4$-Alkylthio;

$R_6$ $C_1$-$C_4$-Alkylthio, Halogen, Nitro oder Amino;

$R_7$ Wasserstoff oder $C_1$-$C_4$-Alkyl;

p 1 oder 2;

oder gegebenenfalls ein Tautomeres davon, jeweils in freier Form oder in Salzform.

(4) Verbindungen der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass, wenn p 1 ist, die Gruppe Ia in 2-, 3-, 6- oder 7-Position des in der Formel I dargestellten, aus den beiden Ringen A und B gebildeten, bicyclischen Ringsystems gebunden ist, mit der weiteren Massgabe, dass, wenn p 2 ist, die Gruppe Ia in 2-, 3-, 7- oder 8-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl und $C_1$-$C_4$-Alkoxy besteht, ausgewählt sind;

wobei bedeuten:

$R_1$ Wasserstoff, $C_1$-$C_4$-Alkyl oder $C_1$-$C_4$-Alkanoyl;

$R_2$, $R_3$, unabhängig voneinander, Wasserstoff oder $C_1$-$C_4$-Alkyl;

$R_4$ Wasserstoff;

$R_5$ $C_1$-$C_4$-Alkyl;

$R_6$ Halogen;

$R_7$ Wasserstoff;
p 1 oder 2;
in freier Form oder in Salzform.

(5) Verbindungen der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass die Gruppe Ia in 2-, 3-, 6- oder 7-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl und $C_1$-$C_4$-Alkoxy besteht, ausgewählt sind;
wobei bedeuten:
$R_1$ Wasserstoff oder $C_1$-$C_4$-Alkanoyl;
$R_2$ Wasserstoff;
$R_3$ $C_1$-$C_4$-Alkyl;
$R_4$ Wasserstoff;
$R_5$ $C_1$-$C_4$-Alkyl;
$R_6$ Halogen;
$R_7$ Wasserstoff;
p 1;
in freier Form oder in Salzform.

(6) Verbindungen der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass die Gruppe Ia in 6- oder 7-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus $C_1$-$C_2$-Alkyl besteht, ausgewählt sind;
wobei bedeuten:
$R_1$ Wasserstoff oder $C_1$-$C_2$-Alkanoyl;
$R_2$ Wasserstoff;
$R_3$ $C_1$-$C_2$-Alkyl;
$R_4$ Wasserstoff;
$R_5$ $C_1$-$C_2$-Alkyl;
$R_6$ Chlor;
$R_7$ Wasserstoff;
p 1;
in freier Form oder in Salzform.

(7) Verbindungen der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder eines dieser anderen (p + 5) substituierbaren Ring-Kohlenstoffatome der beiden Ringe A und B, insbesondere das Ring-Kohlenstoffatom in 2-Position des in der Formel I dargestellten, aus den beiden Ringen A und B gebildeten, bicyclischen Ringsystems, einfach durch $C_1$-$C_2$-Alkyl substituiert ist, mit der Massgabe, dass die Gruppe Ia in 7-Position des in der Formel I dargestellten, aus den beiden Ringen A und B gebildeten, bicyclischen Ringsystems gebunden ist;
wobei bedeuten:
$R_1$ Wasserstoff oder $C_1$-$C_2$-Alkanoyl;
$R_2$ Wasserstoff;
$R_3$ $C_1$-$C_2$-Alkyl;
$R_4$ Wasserstoff;
$R_5$ $C_1$-$C_2$-Alkyl;
$R_6$ Chlor;

$R_7$ Wasserstoff;
p 1;
in freier Form oder in Salzform.

Besonders bevorzugt sind im Rahmen der Erfindung die in den Beispielen H-1 bis H-7.22 genannten Verbindungen der Formel I und gegebenenfalls Tautomere davon, jeweils in freier Form oder in Salzform.

Namentlich bevorzugt sind im Rahmen der Erfindung die folgenden Verbindungen der Formel I:

(a) 2-[1-(5-Chlor-6-ethyl-pyrimidin-4-ylamino)prop-1-yl]-1,2,3,4-tetrahydro-chinolin, (Beispiel H-1);
(b) 2-[1-(5-Chlor-6-ethyl-pyrimidin-4-ylamino)ethyl]-1,2,3,4-tetrahydro-chinolin (Diastereomer 1; Beispiel H-1.1.1);
(c) 2-[1-(5-Chlor-6-ethyl-pyrimidin-4-ylamino)ethyl]-1,2,3,4-tetrahydro-chinolin (Diastereomer 2; Beispiel H-1.1.2);
(d) 2-[1-(5-Chlor-6-ethyl-pyrimidin-4-ylamino)ethyl]-6-methoxy-1,2,3,4-tetrahydro-chinolin (Beispiel H-1.8);
(e) 6-[1-(5-Chlor-6-ethyl-pyrimidin-4-ylamino)ethyl]-1,2,3,4-tetrahydro-chinolin (Beispiel H-3.1);
(f) 7-[1-(5-Chlor-6-ethyl-pyrimidin-4-ylamino)ethyl]-1,2,3,4-tetrahydro-chinolin (Beispiel H-4.1);
(g) das Gemisch aus 6-[1-(5-Chlor-6-ethyl-pyrimidin-4-ylamino)ethyl]-1,2,3,4-tetrahydro-chinolin und 7-[1-(5-Chlor-6-ethyl-pyrimidin-4-ylamino)ethyl]-1,2,3,4-tetrahydro-chinolin (Beispiel H-5.1);
(h) das Gemisch aus 1-Acetyl-6-[1-(5-chlor-6-ethyl-pyrimidin-4-ylamino)ethyl]-1,2,3,4-tetrahydro-chinolin und 1-Acetyl-7-[1-(5-chlor-6-ethyl-pyrimidin-4-ylamino)-ethyl]- 1,2,3,4-tetrahydro-chinolin (Beispiel H-5.5);
(i) das Gemisch aus 1-Acetyl-6-[1-(5-chlor-6-ethyl-pyrimidin-4-ylamino)prop-1-yl]-2-methyl- 1,2,3,4-tetrahydro-chinolin und 1-Acetyl-7-[1-(5-chlor-6-ethyl-pyrimidin-4-ylamino)prop-1-yl]-2-methyl-1,2,3,4-tetrahydro-chinolin (Beispiel H-5.18);
(j) das Gemisch aus 6-[1-(5-Chlor-6-ethyl-pyrimidin-4-ylamino)prop-1-yl]-2-methyl-1,2,3,4-tetrahydro-chinolin und 7-[1-(5-Chlor-6-ethyl-pyrimidin-4-ylamino)prop-1-yl]-2-methyl- 1,2,3,4-tetrahydro-chinolin (Beispiel H-5.21);
(k) 1-Acetyl-8-[1-(5-chlor-6-ethyl-pyrimidin-4-ylamino)ethyl]-2,3,4,5-tetrahydro-1H-benzo[b]azepin (Beispiel H-7.7); und
(l) 1-Acetyl-8-[1-(5-chlor-6-ethyl-pyrimidin-4-ylamino)prop-1-yl] -2,3,4,5-tetrahydro-1H-benzo[b]azepin (Beispiel H-7.8);

jeweils in freier Form oder in Salzform.

Ein weiterer Gegenstand der Erfindung ist das Verfahren zur Herstellung der Verbindungen der Formel I und gegebenenfalls ihrer Tautomeren, jeweils in freier Form oder in Salzform, z. B. dadurch gekennzeichnet, dass man

a) zur Herstellung einer Verbindung der Formel I, worin $R_7$ Wasserstoff, $C_1$-$C_8$-Alkyl oder Benzyl ist, oder gegebenenfalls eines Tautomeren davon, jeweils in freier Form oder in Salzform, eine Verbindung der Formel

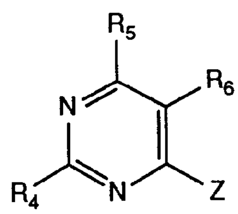

(II),

worin $R_4$, $R_5$ und $R_5$ die für die Formel I angegebenen Bedeutungen haben und Z ein leicht abspaltbarer nucleofuger Rest ist, oder gegebenenfalls ein Tautomeres davon mit einer Verbindung der Formel

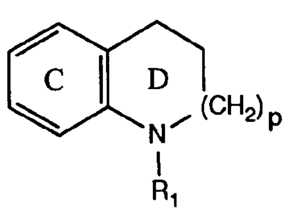

(III),

oder einem Salz davon, vorzugsweise in Gegenwart einer Base, umsetzt und worin $R_1$ und p die für die

Formel I angegebenen Bedeutungen haben und mit einer einzigen Ausnahme der Ring C für den Ring A und der Ring D für den Ring B steht, wobei die Ringe A und B die für die Formel I angegebenen Bedeutungen haben, und wobei die erwähnte Ausnahme darin besteht, dass anstelle der in Formel I erwähnten Gruppe Ia die Gruppe der Formel $H(R_7')N-(C(R_2)(R_3))$ (IIIa) steht, worin $R_7$ Wasserstoff, $C_1$-$C_8$-Alkyl oder Benzyl ist und $R_2$, $R_3$, die für die Formel I angegebenen Bedeutungen haben, oder

b) zur Herstellung einer Verbindung der Formel I, worin $R_7$ die für die Formel I angegebene Bedeutung hat, jedoch von Wasserstoff, $C_1$-$C_8$-Alkyl und Benzyl verschieden ist, oder gegebenenfalls eines Tautomeren davon, jeweils in freier Form oder in Salzform, in eine Verbindung der Formel I, worin $R_7$ Wasserstoff ist, oder gegebenenfalls ein Tautomeres davon, jeweils in freier Form oder in Salzform, den gewünschten, von Wasserstoff, $C_1$-$C_8$-Alkyl und Benzyl verschiedenen, Substituenten $R_7$ durch N-Alkanoylierung, N-Benzoylierung, N-Alkylthiolierung, N-Phenylthiolierung oder N-Benzylthiolierung einführt

und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

Für vor- und nachstehend aufgeführte Ausgangsmaterialien gilt im Hinblick auf deren Tautomere bzw. Salze das vorstehend für Tautomere bzw. Salze von Verbindungen I Gesagte in analoger Weise.

Die vor- und nachstehend beschriebenen Umsetzungen werden in an sich bekannter Weise durchgeführt, z. B. in Ab- oder üblicherweise in Anwesenheit eines geeigneten Lösungs-oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsmediums, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Variante a):

Als Reste Z kommen z. B. in Frage: Fluor, Chlor, Brom, Iod, $C_1$-$C_8$-Alkylthio, wie Methylthio, Ethylthio oder Propylthio, $C_1$-$C_8$-Alkanoyloxy, wie Acetoxy, (Halogen-)$C_1$-$C_8$-Alkansulfonyloxy, wie Methansulfonyloxy, Ethansulfonyloxy oder Trifluormethansulfonyloxy, oder gegebenenfalls substituiertes Phenylsulfonyloxy, wie Benzolsulfonyloxy oder p-Toluolsulfonyloxy, ferner auch Hydroxy.

Geeignete Basen zur Erleichterung der HZ-Abspaltung sind z. B. Alkalimetall- oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, -methanolat, -carbonat, Kalium-tert.-butanolat, -carbonat, Lithiumdiisopropylamid, Kalium-bis-(trimethylsilyl)-amid, Calciumhydrid, Triethylamin, Triethylendiamin, Cyclohexylamin, N-Cyclohexyl-N,N-dimethyl-amin, N,N-Diethylanilin, Pyridin, 4-(N,N-Dimethylamino)pyridin, N-Methylmorpholin, Benzyl-trimethylammoniumhydroxid sowie 1,8-Diaza-bicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethan; Ether, wie Diethylether, tert.-Butylmethylether, Tetrahydrofuran oder Dioxan; Ketone, wie Aceton oder Methylethylketon; Alkohole, wie Methanol, Ethanol, Propanol, Butanol, Ethylenglycol oder Glycerin; Ester, wie Essigsäureethylester oder Essigsäurebutylester; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid. Auch im Ueberschuss eingesetzte Basen, wie Triethylamin, Pyridin, N-Methylmorpholin oder N,N-Diäthylanilin, können als Lösungs- oder Verdünnungsmittel dienen.

Die Umsetzung erfolgt vorteilhaft in einem Temperaturbereich von etwa 0°C bis etwa +180°C, bevorzugt von etwa +20°C bis etwa +130°C, in vielen Fällen bei der Rückflusstemperatur des verwendeten Lösungsmittels.

In einer bevorzugten Ausführungsform der Variante a) wird eine Verbindung II, worin Z Halogen, vorzugs-

weise Chlor, ist, bei Rückflusstemperatur, in einem Alkohol, vorzugsweise in Isopropanol, und in Gegenwart eines Alkylamins, vorzugsweise in Gegenwart von Triethylamin, mit einer Verbindung III umgesetzt.

Die Verbindungen II und gegebenenfalls ihre Tautomeren, jeweils in freier Form oder in Salzform, sind bekannt oder können in Analogie zu bekannten Verbindungen hergestellt werden; entsprechende Angaben finden sich z. B. einerseits bei D. J. Brown, "The Pyrimidines", in "Heterocyclic Compounds" und andererseits in der europäischen Patentanmeldung EP-A-0 470 600.

Die Verbindungen III, in freier Form oder in Salzform, sind zum Teil bekannt oder können in Analogie zu bekannten Verbindungen, z. B. wie nachfolgend beschrieben, hergestellt werden.

a) Beispielsweise kann man Ammoniak oder ein Amin der Formel $H_2NR_7$, (IIIb) in üblicher Weise in Gegenwart eines Reduktionsmittels mit einer Verbindung der Formel IIIc umsetzen, wobei die Verbindungen IIIc den Verbindungen III entsprechen, jedoch anstelle der in den Verbindungen III vorhandenen Gruppe IIIa eine Gruppe der Formel $R_2C(=O)$- (IIId), oder eine Gruppe der Formel $R_3C(=O)$-(IIIe), aufweisen. Geeignete Reduktionsmittel sind z. B. Wasserstoff (in Gegenwart eines Hydrierungskatalysators), Zink/Salzsäure, Natriumcyanoborhydrid, Natriumborhydrid, Eisenpentacarbonyl/alkoholisches Kaliumhydroxid oder Ameisensäure. Man erhält so - durch formalen Ersatz der in den Gruppen IIId, bzw. IIIe, enthaltenen Carbonylfunktion durch ein Wasserstoffatom und eine Gruppe der Formel $H(R_7')N$- (IIIf) - Verbindungen III, die an dem die Gruppe (IIIj) tragenden Kohlenstoffatom mindestens ein Wasserstoffatom aufweisen.

Es kann für diese Umsetzung auch eine - gegebenenfalls vorgängig hergestellte - "Kombinationsverbindung" aus dem Reduktionsmittel und der Verbindung IIIb, z. B. ein entsprechendes Ammoniumformiat oder Formamid, verwendet werden, wobei sich das nach der Umsetzung dieser " Kombinationsverbindung" mit der Verbindung IIIc gegebenenfalls gebildete Primärprodukt mit einer N-formylierten Gruppe IIIj nachfolgend in üblicher Weise hydrolytisch zu einer der gewünschten Verbindungen III N-deformylieren lässt.

In üblicher Weise kann man auch ein gegebenenfalls substituiertes Hydroxylamin mit einer Verbindung IIIc umsetzen, dadurch die Carbonylgruppe in ein gegebenenfalls substituiertes Oxim überführen und dieses zum entsprechenden Amin reduzieren, wobei als Reduktionsmittel z. B. komplexe Metallhydride, wie $LiAlH_4$, Zink/Essigsäure oder Wasserstoff (in Gegenwart eines Hydrierungskatalysators, z. B. in Gegenwart von Raney-Nickel oder Palladium auf Kohle) Verwendung finden. Man erhält so Verbindungen III, die an dem die Gruppe $H(R_7')N$- (IIIf) tragenden Kohlenstoffatom mindestens ein Wasserstoffatom aufweisen und in denen $R_7'$ Wasserstoff ist.

b) Ferner kann man auch in üblicher Weise in Nitroalkylverbindungen der Formel IIIs die Nitroalkylgruppe zur Aminoalkylgruppe reduzieren, wobei die Verbindungen IIIs den Verbindungen III entsprechen, jedoch anstelle der in den Verbindungen III vorhandenen Gruppe IIIa eine Gruppe der Formel $O_2N$-$[C(R_2)(R_3)]$- (IIIg) aufweisen, und wobei als Reduktionsmittel z. B. komplexe Metallhydride, wie $LiAlH_4$, Verwendung finden. Man erhält so Verbindungen III, in denen $R_7'$ Wasserstoff ist.

c) Die obgenannten Reaktionen a) und b) zur Herstellung von Verbindungen der Formel III können auch mit den entsprechenen Chinolin- bzw. Benzo[b]azepin-Derivaten durchgeführt werden, wobei bei der Reduktion, entweder gleichzeitig oder nacheinander, sowohl die Iminogruppe bzw. Nitrogruppe als auch der stickstoffhaltige aromatische Ring-Teil zu den entsprechenden Verbindungen der Formel III reduziert wird.

d) Ferner können Verbindungen der Formel III hergestellt werden, indem acylierte 2-Oxo-1,2,3,4-tetrahydrochinoline bzw. acylierte 2-Oxo-2,3,4,5-tetrahydrobenz[b]azepine wie unter a) und b) beschrieben in die entsprechenden Aminoalkyl-2-oxo-1,2,3,4-tetrahydrochinoline bzw. Aminoalkyl-2-oxo-2,3,4,5-tetrahydrobenz[b]azepine überführt werden und diese anschliessend durch Reduktion mit einem geeigneten Reduktionsmittel, wie z. B. Lithiumaluminiumhydrid oder metallischem Natrium, zu den Verbindungen der Formel III reduziert werden.

Verbindungen III, in denen $R_7'$ Wasserstoff ist, können in üblicher Weise zu Verbindungen III, worin $R_7'$ $C_1$-$C_8$-Alkyl oder Benzyl ist, $C_1$-$C_8$-alkyliert oder benzyliert werden. Dazu setzt man z. B. die Verbindung III, worin $R_7'$ Wasserstoff ist, vorteilhaft in Gegenwart einer Base, z. B. in Gegenwart einer der vorstehend angegebenen Basen, und in einem inerten Lösungs- oder Verdünnungsmittel, z. B. der vorstehend angegebenen Art, mit einer Verbindung der Formel $C_1$-$C_8$-Alkyl-Z (IIIh) oder mit einer Verbindung der Formel Benzyl-Z (IIIi) um, wobei Z jeweils die vorstehend angegebene Bedeutung hat.

Die Verbindungen IIIb, IIIc, IIIh und IIIi sind bekannt oder können in Analogie zu bekannten Verbindungen hergestellt werden.

Beispielsweise können Verbindungen der Formel IIIc hergestellt werden durch Acylierung von entsprechenden nicht acylierten Tetrahydrochinolinen oder Tetrahydrobenzo[b]azepinen; oder durch Acylierung von entsprechenden nicht acylierten Chinolinen bzw. Benzo[b]azepinen und anschliessender Hydrierung des stickstoffhaltigen aromatischen Ring-Teils; gegebenenfalls kann die Carbonylgruppe des Acylrestes vor der Hydrie-

rung in ein Acetal überführt werden, und dieses nach erfolgter Hydrierung des stickstoffhaltigen aromatischen Ring-Teils wieder zur Carbonylgruppe hydrolysiert werden.

Eine weitere Möglichkeit zur Herstellung von Verbindungen der Formel IIIc' besteht darin, dass eine Verbindung der Formel Va, worin $R_2$ und p die angegebenen Bedeutungen haben, und worin R Wasserstoff, $C_1$-$C_8$-Alkyl, $C_1$-$C_4$-Alkoxy-$C_1$-$C_4$-alkyl oder $C_3$-$C_8$-Cycloalkyl bedeutet, zunächst in üblicher Weise zu einer Verbindung der Formel Vb nitriert wird, diese mit Wasserstoff in Gegenwart eines Katalysators zum Anilin der Formel Vc reduziert wird, welches spontan zum Imin Vc' zyklisiert, und dieses mit einem geeigeten Reduktionsmittel, z.B. mit Wasserstoff in Gegenwart eines Katalysators, mit oder ohne Ueberdruck, gegebenenfalls in Gegenwart von Säure, in eine Verbindung der Formel IIIc überführt wird, worin $R_2$, p und R die angegebenen Bedeutungen haben.

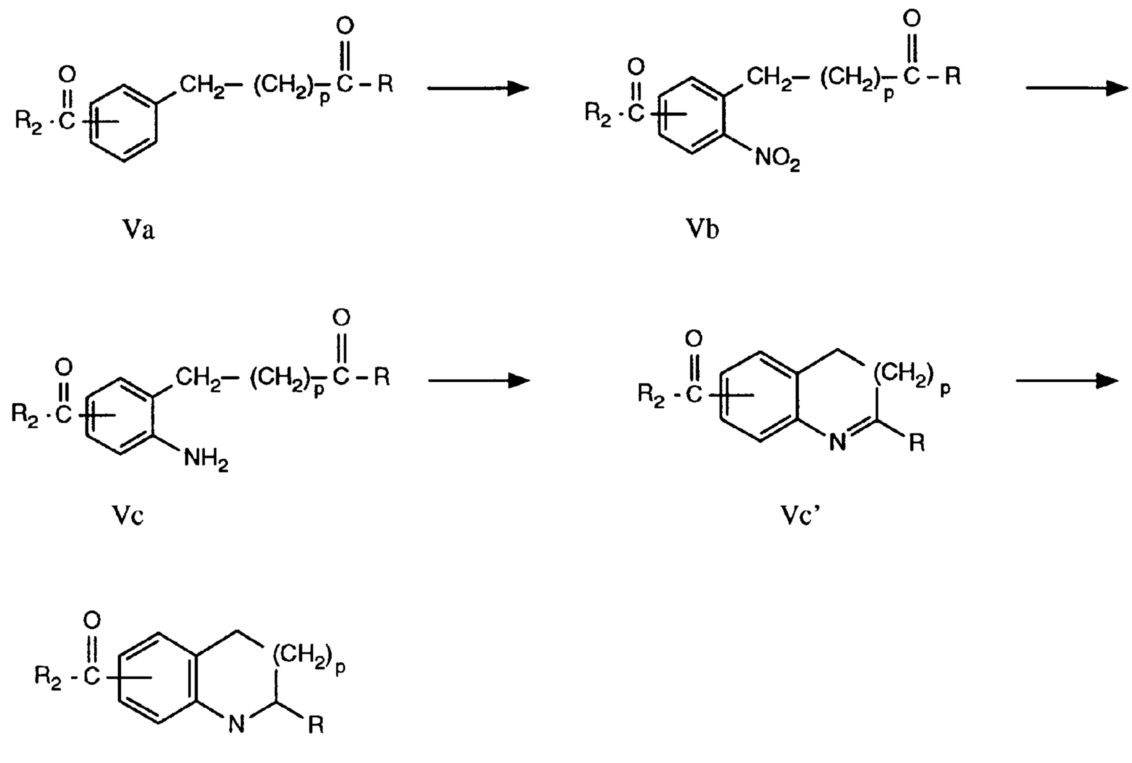

Va Vb

Vc Vc'

IIIc

Variante b):

Die N-Alkanoylierung, N-Benzoylierung, N-Alkylthiolierung, N-Phenylthiolierung oder N-Benzylthiolierung einer Verbindung 1, worin $R_7$ Wasserstoff ist, oder gegebenenfalls eines Tautomeren davon, jeweils in freier Form oder in Salzform, erhältlich beispielsweise gemäss der Verfahrensvariante a), erfolgt in üblicher Weise, z. B. durch Umsetzung einer Verbindung I, worin $R_7$ Wasserstoff ist, oder gegebenenfalls eines Tautomeren davon, jeweils in freier Form oder in Salzform, vorteilhaft in Gegenwart einer Base, z. B. in Gegenwart einer der unter der Variante a) angegebenen Basen, in einem inerten Lösungs-oder Verdünnungsmittel, z. B. in einem inerten Lösungs- oder Verdünnungsmittel der unter der Variante a) angegebenen Art, und in einem Temperaturbereich von etwa -80°C bis etwa +180°C, bevorzugt von etwa 0°C bis etwa +130°C, in vielen Fällen bei der Rückflusstemperatur des verwendeten Lösungsmittels, mit einer Verbindung der Formel $C_1$-$C_8$-Alkanoyl-Z (Ie), mit einer Verbindung der Formel Phenylcarbonyl-Z (If), deren Phenylgruppe unsubstituiert oder durch einen, zwei oder drei Substituenten, ausgewählt aus der Gruppe bestehend aus Halogen und $C_1$-$C_4$-Alkyl, substituiert ist, mit einer Verbindung der Formel $C_1$-$C_8$-Alkylthio-Z (Ig), mit einer Verbindung der Formel Halogen-$C_1$-$C_8$-alkylthio-Z (Ih), mit einer Verbindung der Formel Cyano-$C_1$-$C_8$-alkylthio-Z (Ii), mit einer Verbindung der Formel Phenylthio-Z (Ij) oder mit einer Verbindung der Formel Benzylthio-Z (Ik), wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind.

In den Verbindungen Ie, If, Ig, Ih, Ii, Ij und Ik hat Z jeweils die unter der Variante a) angegebene Bedeutung.

Die Verbindungen Ie, If, Ig, Ih, Ii, Ij und Ik sind bekannt oder können in Analogie zu bekannten Verbindungen hergestellt werden.

Eine verfahrensgemäss oder auf andere Weise erhältliche Verbindung I oder gegebenenfalls ein Tautomeres davon kann in an sich bekannter Weise in eine andere Verbindung I überführt werden, indem man einen oder mehrere Substituenten der Ausgangsverbindung I in üblicher Weise durch (einen) andere(n) erfindungsgemässe(n) Substituenten ersetzt.

Beispielsweise können:

- nicht-halogenhaltige Substituenten bzw. nicht-halogenierte aromatische oder heteroaromatische Ring-Teilstrukturen zu erfindungsgemässen halogenhaltigen Substituenten bzw. halogenierten aromatischen oder heteroaromatischen Ring-Teilstrukturen halogeniert werden;
- Halogensubstituenten gegen andere erfindungsgemässe Substituenten, wie Alkylthio- oder Alkoxy-Substituenten, ausgetauscht werden; insbesondere können
- Alkanoyl- oder Benzoyl-Substituenten $R_1$ gegen Wasserstoff $R_1$ oder
- Wasserstoff $R_1$ gegen Alkyl $R_1$ ausgetauscht werden.

Der besonders bevorzugte Austausch von Alkanoyl- oder Benzoyl-Substituenten $R_1$ gegen Wasserstoff $R_1$ (Deacylierung) erfolgt im allgemeinen in üblicher Weise, kann aber auch unter speziellen Reaktionsbedingungen, beispielsweise durch Umsetzung der acylierten Verbindung I mit elementarem Natrium in einem höheren sekundären Alkohol, wie Cyclohexanol, unter erhöhter Temperatur, z. B. in einem Temperaturbereich zwischen 50°C und 200°C, erfolgen.

Es ist dabei, je nach Wahl der dafür jeweils geeigneten Reaktionsbedingungen und Ausgangsmaterialien, möglich, in einem Reaktionsschritt nur einen Substituenten durch einen anderen erfindungsgemässen Substituenten zu ersetzen, oder es können in demselben Reaktionschritt mehrere Substituenten durch andere erfindungsgemässe Substituenten ersetzt werden. Beispielsweise können gleichzeitig zwei oder mehrere gleiche Halogensubstituenten in denselben oder, sofern vorhanden, in verschiedene Substituenten und/oder Ringe eingeführt werden. Ebenso kann z. B. ein bereits durch einen bestimmten Substituenten, z. B. Chlor, beispielsweise einfach, substituierter Substituent oder Ring - im Rahmen der erfindungsgemässen Definitionen der Variablen - durch einen oder mehrere weitere gleichartige Substituenten zusätzlich substituiert, z. B. chloriert, werden, d. h. beispielsweise in den di- oder einen noch höher-substituierten, z. B. -chlorierten, Zustand überführt werden.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Die Verbindungen I und gegebenenfalls ihre Tautomeren, jeweils in freier Form oder in Salzform, können teilweise in Form eines der möglichen Isomeren oder als Gemisch derselben, z. B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen I und gegebenenfalls ihrer Tautomeren, jeweils in freier Form oder in Salzform, können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird, oder durch Überführung in diastereomere Salze, z. B. durch Umsetzung eines basischen Endstoffracemats mit einer optisch aktiven Säure, wie Carbonsäure, z. B. Campher-, Wein- oder Apfelsäure, oder Sulfonsäure, z. B. Camphersulfonsäure, und Trennung des auf diese Weise erhaltenen Diastereomerengemisches, z. B. auf Grund ihrer verschiedenen Löslichkeiten durch fraktionierte Kristallisation, in die Diastereomeren, aus denen das gewünschte Enantiomere durch Einwirkung geeigneter, z. B. basischer, Mittel freigesetzt werden kann.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere, oder Isomerengemisch, z. B. Enantiomerengemisch, sofern die einzelnen Komponenten unterschiedliche biologische Wirksamkeit besitzen.

Die Verbindungen I und gegebenenfalls ihre Tautomeren, jeweils in freier Form oder in Salzform, können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen.

Die Erfindung betrifft insbesondere die in den Beispielen beschriebenen Herstellungsverfahren.

Erfindungsgemäss für die Herstellung der Verbindungen I bzw. ihrer Salze verwendete Ausgangsstoffe und Zwischenprodukte, jeweils in freier Form oder in Salzform, die neu sind, ihre Verwendung und Verfahren zu ihrer Herstellung bilden ebenfalls einen Gegenstand der Erfindung.

In diesem Zusammenhang sind die Verbindungen III von spezieller Bedeutung, welche einen weiteren Gegenstand der vorliegenden Erfindung bilden, ebenso wie ihre Herstellung und ihre Verwendung als Zwischenprodukte.

In 4-Position aminosubstituierte Pyrimidine sind bereits bekannt, z. B. aus der europäischen Patentanmeldung mit der Veröffentlichungsnummer 0126 254. Von diesen bekannten Verbindungen unterscheiden sich die Verbindungen I der vorliegenden Erfindung strukturell in charakteristischer Weise; zudem zeigen die Verbindungen I der vorliegenden Erfindung eine unerwartet hohe mikrobizide, insektizide und akarizide Wirksamkeit.

Die Verbindungen I und gegebenenfalls ihre Tautomeren, jeweils in freier Form oder in Salzform, der vorliegenden Erfindung sind bei Raumtemperatur stabile Oele, Harze oder Feststoffe. Sie lassen sich auf dem Agrarsektor und verwandten Gebieten präventiv und/oder kurativ als Wirkstoffe bei der Bekämpfung von pflanzenschädigenden Mikroorganismen und von Schädlingen des akariziden Typus einsetzen. Die erfindungsgemässen Wirkstoffe der Formel I zeichnen sich - auch bei niedrigen Anwendungskonzentrationen - nicht nur durch hervorragende Wirkung, sondern auch durch gute Pflanzenverträglichkeit aus.

Die erfindungsgemässen Wirkstoffe der Formel I weisen ein für praktische Bedürfnisse sehr günstiges biozides Spektrum zur Bekämpfung von Schädlingen der Ordnung Akarina und der Klasse Insecta und von phytopathogenen Mikroorganismen, insbesondere Fungi, auf. Sie besitzen sehr vorteilhafte, insbesondere systemische, Eigenschaften und werden zum Schutz von zahlreichen Kulturpflanzen eingesetzt. Mit den Wirkstoffen der Formel I können an Pflanzen oder Pflanzenteilen (Früchten, Blüten, Laubwerk, Stengeln, Knollen, Wurzeln) von unterschiedlichen Nutzkulturen die auftretenden Schädlinge eingedämmt oder vernichtet werden, wobei auch später zuwachsende Pflanzenteile, z. B. von phytopathogenen Mikroorganismen, verschont bleiben.

Verbindungen I sind z. B. gegen die den folgenden Klassen angehörenden phytopathogenen Pilze wirksam: Fungi imperfecti (z. B. Botrytis, Pyricularia, Helminthosporium, Fusarium, Septoria, Cercospora und Alternaria) und Basidiomyceten (z. B. Rhizoctonia, Hemileia, Puccinia). Darüber hinaus wirken sie gegen die Klassen der Ascomyceten (z. B. Venturia und Erysiphe, Podosphaera, Monilinia, Uncinula) und der Oomyceten

(z. B. Phytophthora, Pythium, Plasmopara).

Die Verbindungen I können ferner als Beizmittel zur Behandlung von Saatgut (Früchten, Knollen, Körnern) und Pflanzenstecklingen zum Schutz vor Pilzinfektionen sowie gegen im Erdboden auftretende phytopathogene Pilze eingesetzt werden.

Die erfindungsgemässen Verbindungen I sind zudem wertvolle Wirkstoffe bei der Bekämpfung von Schädlingen der Ordnung Akarina und der Klasse Insecta an Nutz- und Zierpflanzen in der Landwirtschaft, insbesondere in Baumwoll-, Gemüse- und Obstpflanzungen, im Forst, im Vorrats- und Materialschutz-Bereich sowie im Hygienesektor, insbesondere an Haus- und Nutztieren. Sie sind gegen verschiedene Entwicklungsstadien wirksam. Dabei kann sich ihre Wirkung in unmittelbarer Abtötung der Schädlinge oder erst nach einiger Zeit, beispielsweise bei einer Häutung, oder in einer deutlich verminderten Eiablage und/oder Schlupfrate zeigen. Zur Ordnung Akarina gehören z. B. Boophilus spp. und Tetranychus spp.; diese Aufzählung ist nicht limitierend.

Die Erfindung betrifft auch die Mittel, die als Wirkstoff Verbindungen I enthalten, insbesondere pflanzenschützende Mittel, sowie deren Verwendung auf dem Agrarsektor und verwandten Gebieten. Darüber hinaus schliesst die Erfindung auch die Herstellung dieser Mittel ein, die gekennzeichnet ist durch das innige Vermischen der Aktivsubstanz mit einer oder mehreren der nachstehend beschriebenen Substanzen bzw. Substanzgruppen. Eingeschlossen in die Erfindung ist auch ein Verfahren zur Behandlung von Pflanzen, das sich durch Applikation der neuen Verbindungen I bzw. der neuen Mittel auszeichnet.

Als Zielkulturen für den pflanzenschützenden Einsatz gelten im Rahmen der Erfindung beispielsweise folgende Pflanzenarten: Getreide (Weizen, Gerste, Roggen, Hafer, Reis, Mais, Sorghum und verwandte Spezies); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Campfer) und Pflanzen wie Tabak, Nüsse, Kaffee, Eierfrüchte, Zuckerrohr, Tee, Pfeffer, Reben, Hopfen, Bananen- und Naturkautschukgewächse sowie Zierpflanzen.

Wirkstoffe I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können z. B. Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und sind in der Formulierungstechnik zweckdienliche Stoffe, z. B. natürliche oder regenerierte mineralische Stoffe, Lösungs-, Dispergier-, Netz-, Haft-, Verdickungs-, Binde- oder Düngemittel.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Bei Wasserreiskulturen kann man solche Granulate dem überfluteten Reisfeld zudosieren. Die Verbindungen I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen I werden dabei in unveränderter Form oder vorzugsweise zusammen mit den in der Formulierungstechnik üblichen Hilfsmitteln eingesetzt. Dafür werden sie zweckmässigerweise z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln oder Granulaten, z. B. durch Verkapselung in, z. B. polymeren, Stoffen, in bekannter Weise verarbeitet Die Anwendungsverfahren wie Versprühen, Vernebeln, Verstäuben, Verstreuen, Bestreichen oder Giessen werden ebenso wie die Art der Mittel den angestrebten Zielen und den gegebenen Verhältnissen entsprechend gewählt.

Günstige Aufwandmengen liegen im allgemeinen bei 5 g bis 2 kg Aktivsubstanz (AS) je Hektar (ha), bevorzugt bei 10 g bis 1 kg AS/ha, insbesondere bei 20 g bis 600 g AS/ha.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen des Wirkstoffs mit Streckmitteln, wie Lösungsmitteln, festen Trägerstoffen und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glycole sowie deren Ether und Ester, wie Ethanol, Ethylenglycol, Ethylenglycolmonomethyl- oder -ethylether, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, und Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht-sorptive Trägermaterialien z. B. Calcit oder Sand in Frage. Darüber hinaus kann eine Vielzahl von vorgranulierten Materialien anorganischer oder organischer Natur, wie Dolomit oder zerkleinerte Pflanzenrückstände, verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglycolether, Polypropylen-/Polyethylen-oxidaddukte, Tributylphenoxypolyethylenethanol, Polyethylenglycol und Octylphenoxypolyethoxyethanol erwähnt.

Ferner kommen auch Fettsäureester von Polyoxyethylensorbitan, wie das Polyoxyethylensorbitantrioleat, in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gewichtsprozent, insbesondere 0,1 bis 95 Gewichtsprozent, Wirkstoff der Formel I, 99,9 bis 1 Gewichtsprozent, insbesondere 99,8 bis 5 Gewichtsprozent, eines festen oder flüssigen Zusatzstoffes und 0 bis 25 Gewichtsprozent, insbesondere 0,1 bis 25 Gewichtsprozent, eines Tensids.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel oder Haftmittel, sowie Dünger oder andere Wirkstoffe zur Erzielung spezieller Effekte enthalten.

Die nachfolgenden Beispiele illustrieren die vorstehend beschriebene Erfindung, ohne dieselbe in ihrem Umfang in irgendeiner Weise einzuschränken. Temperaturen sind in Celsiusgraden angegeben. Die folgenden Abkürzungen werden verwendet: Ac = Acetyl; Et=Ethyl; Iso-Pr=Isopropyl; Me=Methyl; Ph=Phenyl; Pr=n-Propyl; Smp.=Schmelzpunkt. DS=Diastereomer; Reg = Regioisomer. "NMR" steht für "Kernmagnetisches Resonanzspektrum". MS=Massenspektrum. " %" steht für "Gewichts-prozent", sofern entsprechende Konzentrationen nicht in einer anderen Einheit angegeben sind. Unter dem Begriff "erfindungsgemässer Wirkstoff" ist jeweils eine Verbindung I oder gegebenenfalls ein Tautomeres davon, jeweils in freier Form oder in Form eines agrochemisch verwendbaren Salzes, zu verstehen.

Herstellungsbeispiele für erfindungsgemässe Verbindungen

Beispiel H-1:

In einem Sulfierkolben werden bei Raumtemperatur 1,3g 2-(1-Amino-propyl)-1,2,3,4-tetrahydrochinolin, 1,4g 4,5-Dichlor-6-ethylpyrimidin und 0,85g Triethylamin in 60ml absolutem Isopropanol vorgelegt. Die Innentemperatur des Kolbens wird auf 80 C erhöht und das Reaktionsgemisch 12Stunden unter Rückfluss gerührt. Das Lösungsmittel wird sodann im Wasserstrahlvakuum entfernt, der Rückstand in einem Gemisch aus 200ml Essigsäureethylester und 70ml Wasser aufgenommen und die Mischung gut geschüttelt. Die organische Phase wird abgetrennt und die wässrige Phase noch 2x mit jeweils 100ml Essigester extrahiert. Die organische Phase wird über $Na_2SO_4$ getrocknet und das Lösungsmittel im Wasserstrahlvakuum abgezogen. Das zurückbleibende Rohprodukt kann anschliessend durch Flashchromatographie an Kieselgel (FLiessmittel: Essigsäureethylester/n-Hexan 1:3) weiter gereinigt werden. Man erhält 1,6g 2-[1-(5-Chlor-6-ethylpyrimidin-4-ylamino)propyl]-1,2,3,4-tetrahydrochinolin in Form eines roten Oels ($n_D^{50}$ = 1,5822).

Beispiel H-2:

Ein Regioisomerengemisch aus 4,5g 1-Acetyl-6-[1-(5-Chlor-6-ethylpyrimidin-4-ylamino)ethyl]-1,2,3,4-tetrahydrochinolin und 1-Acetyl-7[1-(5-Chlor-6-ethylpyrimidin-4-ylamino)ethyl]-1,2,3,4-tetrahydrochinolin wird bei Raumtemperatur in 45ml ca. 4,5N ethanolischer Clorwasserstofflösung gelöst und anschliessend unter Rühren 8 Stunden am Rückfluss gehalten. Das Lösungsmittel wird sodann im Wasserstrahlvakuum entfernt und der Rückstand in ca. 30ml Wasser gelöst. Anschliessend wird die Lösung mit kalter 1N Natriumhydroxidlösung auf pH 8 gestellt und das Gemisch mehrfach mit Diethylether extrahiert. Die vereinigten organischen Phasen werden über $Na_2SO_4$ getrocknet und im Wasserstrahlvakuum eingedampft. Das Rohprodukt kann anschliessend durch Flashchromatographie an Kieselgel (Fliessmittel: Essigsäureethylester/n-Hexan 3:1) gereinigt werden. Man erhält 3,2g eines Regioisomerengemisches aus 6-[1-(5-Chlor-6-ethylpyrimidin-4-ylamino)ethyl]-1,2,3,4-tetrahydrochinolin und 7-[1-(5-Chlor-6-ethylpyrimidin-4-ylamino)ethyl]-1,2,3,4-tetrahydrochinolin (Verhältnis ca. 1:1 aus $^1$H-NMR-Integration) in Form eines roten Oels ($^1$H-NMR).

In analoger Weise wie in den Beispielen H-1 und H-2 beschrieben oder mittels einer entsprechenden anderen der vorstehend angegebenen Verfahrensweisen können auch die in den nachfolgenden Tabellen aufgeführten Verbindungen hergestellt werden.

Tabelle 1: Verbindungen der Formel I.1

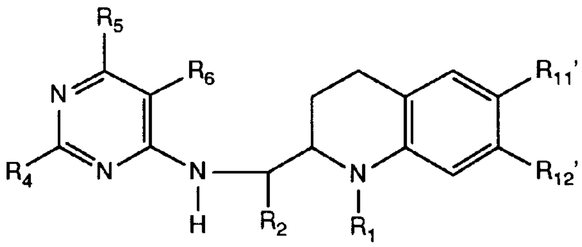

(I.1)

| Bsp. H | $R_1$ | $R_2$ | $R_4$ | $R_5$ | $R_6$ | $R_{11}'$ | $R_{12}'$ | Phys. Daten Smp. C |
|---|---|---|---|---|---|---|---|---|
| 1.1.1 | H | Me | H | Et | Cl | H | H | 124-125 (DS 1) |
| 1.1.2 | H | Me | H | Et | Cl | H | H | 134-136 (DS 2) |
| 1.2 | H | Me | H | Et | Br | H | H | |
| 1.3 | H | Pr | H | Me | Cl | H | H | |
| 1.4 | H | 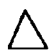 | | H | Me | Cl | H | |
| 1.5 | H | Me | H | Me | Cl | H | H | |
| 1.6 | H | Et | H | Et | Br | H | H | Oel |
| 1.7 | H | Et | H | Me | Cl | H | H | |
| 1.8 | H | Me | H | Et | Cl | OMe | H | 79-84 |
| 1.9 | H | Me | H | Me | Cl | OMe | H | |
| 1.10 | H | Me | H | Me | Br | OMe | H | |
| 1.11 | H | Me | H | Et | Cl | H | Cl | |
| 1.12 | H | Me | H | Et | Br | H | Cl | |
| 1.13 | H | Me | H | Me | Cl | H | Cl | |
| 1.14 | H | Et | H | Et | Cl | OMe | H | |
| 1.15 | H | Et | H | Me | Cl | OMe | H | |
| 1.16 | H | Et | H | Et | Cl | H | Cl | |
| 1.17 | H | Me | H | Et | Br | Br | H | |
| 1.18 | H | Me | H | Me | Cl | Br | H | |
| 1.19 | H | Me | H | Me | Br | Br | H | |
| 1.20 | H | Me | H | Et | $NH_2$ | H | H | |
| 1.21.1 | H | Me | H | Et | Cl | Cl | H | 148-154 (DS 1) |

| Bsp. H | $R_1$ | $R_2$ | $R_4$ | $R_5$ | $R_6$ | $R_{11}$' | $R_{12}$' | Phys. Daten Smp. C |
|---|---|---|---|---|---|---|---|---|
| 1.21.2 | H | Me | H | Et | Cl | Cl | H | 118-120 (DS 2) |
| 1.22 | H | Et | H | Et | Cl | Cl | H | Harz, $^1$H-NMR (nur 1 DS) |
| 1.23 | H | Me | Me | Et | Cl | H | H | |
| 1.24.1 | H | Me | H | Et | Cl | F | H | 108-110 (DS 1) |
| 1.24.2 | H | Me | H | Et | Cl | F | H | 120-126 (DS 2) |
| 1.25 | H | Et | H | Et | Cl | F | H | |
| 1.26.1 | Ac | Me | H | Et | Cl | H | H | Harz, $^1$H-NMR (DS 1) |
| 1.26.2 | Ac | Me | H | Et | Cl | H | H | 109-113 (DS 2) |
| 1.27 | H | Pr | H | Et | Cl | Me | H | Oel, $n_D^{50}$ = 1,5603 |
| 1.28 | Me | Me | H | Et | Cl | H | H | |
| 1.29 | Me | Et | H | Et | Cl | H | H | |
| 1.30 | H | Me | H | Et | Cl | O-Ph | H | |
| 1.31 | H | Et | H | Et | Cl | O-Ph | H | |
| 1.32 | H | Me | H | Et | Cl | 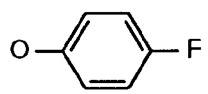 | H | |
| 1.33 | H | Et | H | Et | Cl | 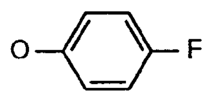 | H | |
| 1.34 | H | Et | H | Et | Cl | 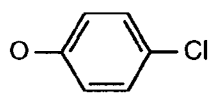 | H | |
| 1.35.1 | H | Me | H | Et | Cl | 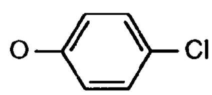 | H | 82-85 (DS 1) |
| 1.35.2 | H | Me | H | Et | Cl | 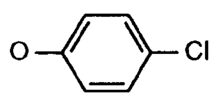 | H | Oel, $^1$H-NMR |
| 1.36 | H | Me | H | Me | Cl | 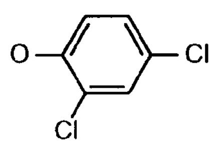 | H | |
| 1.37 | H | Me | H | Me | Cl | 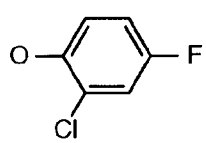 | H | |

| Bsp. H | $R_1$ | $R_2$ | $R_4$ | $R_5$ | $R_6$ | $R_{11}$' | $R_{12}$' | Phys. Daten Smp. C |
|---|---|---|---|---|---|---|---|---|
| 1.38 | H | Me | H | Et | Cl | 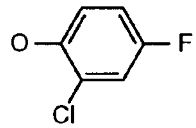 | H | |
| 1.39 | H | Me | Me | Et | Cl | 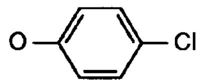 | H | |
| 1.40 | H | Me | Me | Et | Cl | 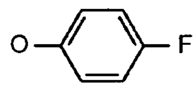 | H | |
| 1.41 | $SO_2Me$ | Me | H | Et | Cl | H | H | |
| 1.42 | $SO_2Ph$ | Me | H | Me | Cl | H | H | |
| 1.43 | H | Me | H | Et | Cl | $OCF_2H$ | H | |
| 1.44 | H | Me | H | Et | Cl | $OCF_3$ | H | |
| 1.45 | H | Et | H | Et | Cl | $OCF_2H$ | H | |
| 1.46 | H | Et | H | Et | Cl | $OCF_3$ | H | |

Tabelle 2: Verbindungen der Formel I.2

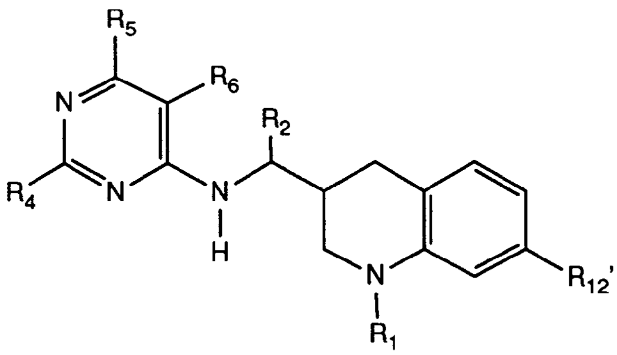

(I.2)

| Beispiel | $R_1$ | $R_2$ | $R_4$ | $R_5$ | $R_6$ | $R_{12}'$ | Phys. Daten |
|---|---|---|---|---|---|---|---|
| H-2.1.1 | H | Me | H | Et | Cl | H | Smp. 109-112 C (DS 1) |
| H-2.1.2 | H | Me | H | Et | Cl | H | Oel; $^1H$-NMR |
| H-2.2 | H | Me | H | Et | Br | H | |
| H-2.3 | H | Me | H | Me | Cl | H | |
| H-2.4 | H | Me | H | Et | Cl | Cl | |
| H-2.5 | H | Me | H | Et | Br | Cl | |
| H-2.6 | H | Me | H | Me | Cl | Cl | |
| H-2.7 | Ac | Me | H | Et | Cl | H | |
| H-2.8 | Me | Me | H | Et | Cl | H | |
| H-2.9 | H | Me | Me | Et | Cl | H | |
| H-2.10 | $SO_2Me$ | Me | H | Et | Cl | H | |
| H-2.11 | $SO_2Ph$ | Me | H | Et | Cl | H | |
| H-2.12 | H | Et | H | Et | Cl | H | |
| H-2.13 | H | 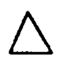 | H | Et | Cl | H | |
| H-2.14 | Me | Et | H | Et | Cl | H | |
| H-2.15 | Me | Me | H | Et | Cl | H | |
| H-2.16 | Ac | Et | H | Et | Cl | H | |
| H-2.17 | H | Me | H | $CH_2OMe$ | Cl | H | |
| H-2.18 | H | Et | H | $CH_2OMe$ | Cl | H | |

Tabelle 3: Verbindungen der Formel I.3

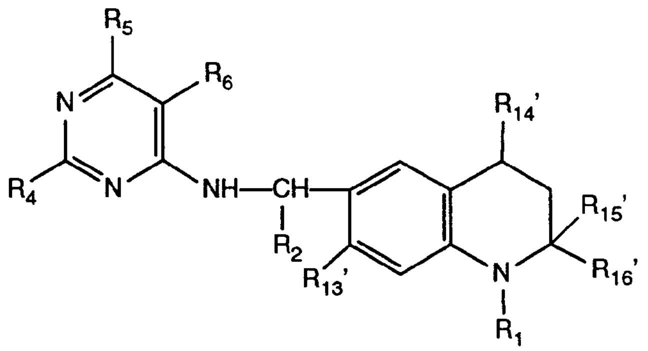

(I.3)

| Beispiel | $R_1$ | $R_2$ | $R_4$ | $R_5$ | $R_6$ | $R_{13}'$ | $R_{14}'$ | $R_{15}'$ | $R_{16}'$ | Phys. Daten |
|---|---|---|---|---|---|---|---|---|---|---|
| H-3.1 | H | Me | H | Et | Cl | H | H | H | H | Oel; $n_D^{50} = 1,6002$ |
| H-3.2 | H | Et | H | Et | Cl | Me | Me | Me | Me | Oel, $^1$H-NMR |
| H-3.3 | H | Me | H | Et | Cl | Me | Me | Me | Me | Oel, $n_D^{50} = 1,5658$ |
| H-3.4 | Et | Me | H | Et | Cl | H | H | H | H | |
| H-3.5 | Et | Et | H | Et | Cl | H | H | H | H | |
| H-3.6 | Me | Me | H | Et | Cl | H | H | H | H | |
| H-3.7 | Me | Et | H | Et | Cl | H | H | H | H | |
| H-3.8 | Ac | Me | H | Et | Cl | H | H | H | H | |
| H-3.9 | Ac | Et | H | Et | Cl | H | H | H | H | |
| H-3.10 | H | 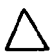 | H | Et | Cl | H | H | H | H | |
| H-3.11 | H | Me | H | Et | Br | H | H | H | H | |
| H-3.12 | H | Me | H | Me | Br | H | H | H | H | |
| H-3.13 | Ac | Me | H | Et | Cl | Me | Me | Me | Me | Smp. 52-55°C |
| H-3.14.1 | Ac | Et | H | Et | Cl | Me | Me | Me | Me | Smp. 57-60°C (DS 1) |
| H-3.14.2 | Ac | Et | H | Et | Cl | Me | Me | Me | Me | Smp. 151-153°C (DS 2) |
| H-3.15 | H | Me | H | Et | Cl | H | H | H | Me | |
| H-3.16 | H | Me | H | Me | Cl | H | H | H | Me | |
| H-3.17 | H | Et | H | Me | Cl | H | H | H | Me | |
| H-3.18 | H | Et | H | Et | Cl | H | H | H | Me | |
| H-3.19 | Ac | Me | H | Me | Cl | H | H | H | Me | |

| Beispiel | $R_1$ | $R_2$ | $R_4$ | $R_5$ | $R_6$ | $R_{13}$' | $R_{14}$' | $R_{15}$' | $R_{16}$' | Phys. Daten |
|---|---|---|---|---|---|---|---|---|---|---|
| H-3.20 | Ac | Me | H | Et | Cl | H | H | H | Me | |
| H-3.21 | Ac | Et | H | Me | Cl | H | H | H | Me | |
| H-3.22 | Ac | Et | H | Et | Cl | H | H | H | Me | |
| H-3.23 | Me | Me | H | Et | Cl | H | H | H | Me | |
| H-3.24 | Me | Et | H | Et | Cl | H | H | H | Me | |
| H-3.25 | H | Et | H | Et | Cl | H | H | H | H | Harz, $^1$H-NMR |
| H-3.26 | H | Et | H | Me | Cl | H | H | H | H | |

Tabelle 4: Verbindungen der Formel I.4

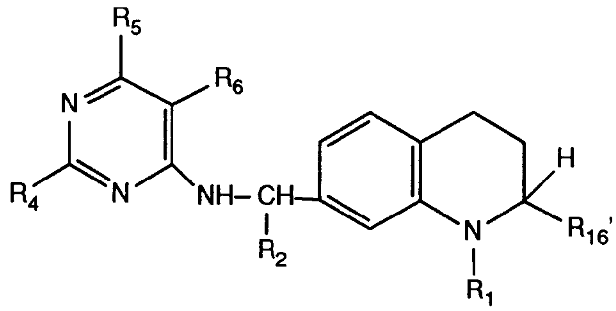

(I.4)

| Beispiel | $R_1$ | $R_2$ | $R_4$ | $R_5$ | $R_6$ | $R_{16}'$ | Phys. Daten |
|---|---|---|---|---|---|---|---|
| H-4.1 | H | Me | H | Et | Cl | H | Oel, $n_D^{50} = 1,5882$ |
| H-4.2 | H | Me | H | Me | Cl | H | Oel |
| H-4.3 | H | Me | H | Me | Br | H | |
| H-4.4 | H | Et | H | Et | Cl | H | Oel |
| H-4.5 | H | Et | H | Me | Cl | H | |
| H-4.6 | H | Et | H | Me | Br | H | |
| H-4.7 | H | Me | H | Et | Cl | Me | |
| H-4.8 | H | Me | H | Me | Cl | Me | |
| H-4.9 | H | Me | H | Me | Br | Me | |
| H-4.10 | H | Et | H | Et | Cl | Me | |
| H-4.11 | H | Et | H | Me | Cl | Me | |
| H-4.12 | H | Et | H | Me | Br | Me | |
| H-4.13 | Ac | Me | H | Et | Cl | H | Smp. 96-98°C |
| H-4.14 | Ac | Et | H | Et | Cl | H | |
| H-4.15 | Ac | Me | H | Et | Cl | Me | |
| H-4.16 | Ac | Et | H | Et | Cl | Me | |
| H-4.17 | Ac | 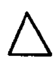 | H | Et | Cl | Me | |
| H-4.18 | CO-Et | Me | H | Et | Cl | Me | Oel, $^1$H-NMR |
| H-4.19 | Me | Me | H | Et | Cl | H | |
| H-4.20 | Me | Me | H | Et | Cl | Me | Oel, $^1$H-NMR |
| H-4.21 | Me | Et | H | Et | Cl | Me | |
| H-4.22 | H | Me | Me | Et | Cl | Me | |
| H-4.23 | H | Me | H | Et | Br | Me | |

| Beispiel | $R_1$ | $R_2$ | $R_4$ | $R_5$ | $R_6$ | $R_{16}$' | Phys. Daten |
|---|---|---|---|---|---|---|---|
| H-4.24 | Et | Me | H | Et | Cl | Me | Oel, $^1$H-NMR |
| H-4.25 | H | Me | H | Et | Cl | 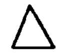 | |
| H-4.26 | $SO_2CH_3$ | Me | H | Et | Cl | H | |
| H-4.27 | CO-Et | Me | H | Et | Cl | H | Oel, $^1$H-NMR |
| H-4.28 | CO-Pr | Me | H | Et | Cl | H | |
| H-4.29 | CO-Et | Et | H | Et | Cl | H | |
| H-4.30 | CO-Pr | Et | H | Et | Cl | H | |
| H-4.31 | CO-Pr | Me | H | Et | Cl | Me | |
| H-4.32 | CO-Et | Et | H | Et | Cl | Me | |
| H-4.33 | H | Me | H | $CH_2OCH_3$ | Cl | H | |
| H-4.34 | H | Et | H | $CH_2OCH_3$ | Cl | H | |
| H-4.35 | H | Me | H | $CH_2OCH_3$ | Cl | Me | |
| H-4.36 | H | Et | H | $CH_2OCH_3$ | Cl | Me | |
| H-4.37 | Et | Et | H | Et | Cl | Me | |

Tabelle 5: Verbindungsgemische der Formeln (I.4) und (I.5)

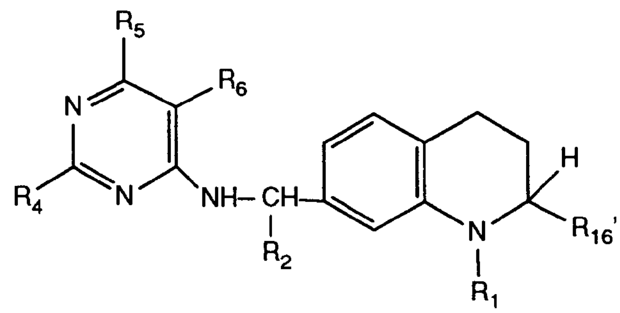

(I.4)

und

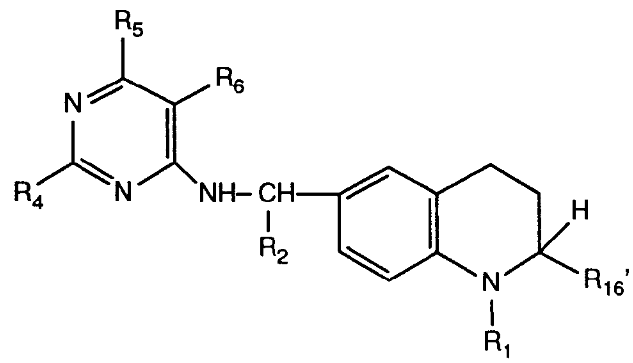

(I.5)

| Beispiel | $R_1$ | $R_2$ | $R_4$ | $R_5$ | $R_6$ | $R_{16}$' | Phys. Daten |
|---|---|---|---|---|---|---|---|
| H-5.1 | H | Me | H | Et | Cl | H | Oel |
| H-5.2 | H | Me | H | Et | Br | H | |
| H-5.3 | H | Me | H | Me | Cl | H | |
| H-5.4 | H | Me | H | Me | Br | H | |
| H-5.5 | Ac | Me | H | Et | Cl | H | Oel, $^1$H-NMR |
| H-5.6 | Ac | Me | H | Et | Br | H | |
| H-5.7 | Ac | Me | H | Me | Cl | H | |
| H-5.8 | H | Et | H | Et | Cl | H | Oel, $n_D^{50}$ = 1.5906 |
| H-5.9 | H | Et | H | Et | Br | H | |
| H-5.10 | H | Et | H | Me | Cl | H | |
| H-5.11 | Ac | Et | H | Et | Cl | H | Oel, $n_D^{50}$ = 1.5720 |
| H-5.12 | Ac | Et | H | Me | Cl | H | |
| H-5.13 | H | Me | H | Et | Cl | Me | Oel, $^1$H-NMR |
| H-5.14 | H | Me | H | Et | Br | Me | |
| H-5.15 | H | Me | H | Me | Cl | Me | |
| H-5.16 | H | Me | H | Me | Br | Me | |

| Beispiel | $R_1$ | $R_2$ | $R_4$ | $R_5$ | $R_6$ | $R_{16}$' | Phys. Daten |
|---|---|---|---|---|---|---|---|
| H-5.17 | H | Me | H | 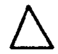 | Cl | Me | |
| H-5.18 | Ac | Et | H | Et | Cl | Me | Oel, $n_D^{50} = 1.5634$ |
| H-5.19 | Ac | Et | H | Et | Br | Me | |
| H-5.20 | Ac | Et | H | Me | Cl | Me | |
| H-5.21 | H | Et | H | Et | Cl | Me | Oel, $n_D^{50} = 1.5770$ |
| H-5.22 | H | Et | H | Me | Cl | Me | |
| H-5.23 | Me | Me | H | Et | Cl | Me | |
| H-5.24 | Me | Et | H | Et | Cl | Me | |
| H-5.25 | Me | 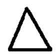 | H | Et | Cl | Me | |
| H-5.26 | Me | Et | H | Me | Cl | Me | |
| H-5.27 | H | Et | H | Et | Cl | 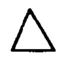 | |
| H-5.28 | CO-Et | Me | H | Et | Cl | Me | |
| H-5.29 | CO-Et | Et | H | Et | Cl | Me | |
| H-5.30 | CO-Et | Me | H | Et | Cl | H | |
| H-5.31 | CO-Et | Et | H | Et | Cl | H | |
| H-5.32 | CHO | Me | H | Et | Cl | H | |
| H-5.33 | CHO | Me | H | Et | Cl | Me | |
| H-5.34 | $SO_2Me$ | Me | H | Et | Cl | H | |
| H-5.35 | $SO_2Me$ | Me | H | Me | Cl | H | |
| H-5.36 | $SO_2Ph$ | Me | H | Et | Cl | H | |
| H-5.37 | H | Me | Me | Et | Cl | H | |
| H-5.38 | H | Et | Me | Et | Cl | H | |
| H-5.39 | H | Et | Me | Et | Cl | H | |
| H-5.40 | H | Me | Me | Me | Cl | Me | |
| H-5.41 | H | Et | Me | Et | Cl | Me | |
| H-5.42 | H | Me | H | $CH_2OMe$ | Cl | H | |
| H-5.43 | H | Et | H | $CH_2OMe$ | Cl | H | |
| H-5.44 | H | Me | H | $CH_2OMe$ | Cl | Me | |
| H-5.45 | H | Et | H | $CH_2OMe$ | Cl | Me | |
| H-5.46 | Me | Me | H | $CH_2OMe$ | Cl | Me | |
| H-5.47 | Me | Et | H | $CH_2OMe$ | Cl | Me | |

Tabelle 6: Verbindungen der Formel I.6

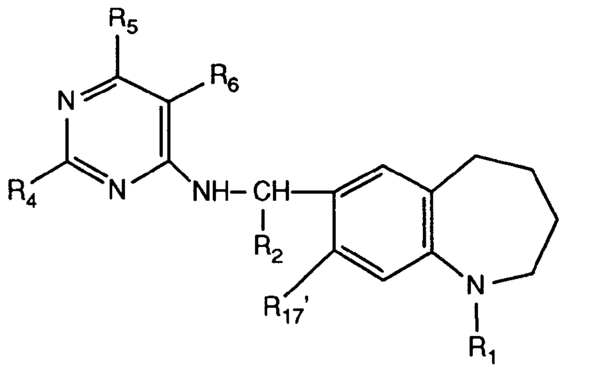

(I.6)

| Beispiel | $R_1$ | $R_2$ | $R_4$ | $R_5$ | $R_6$ | $R_{17}'$ | Phys. Daten |
|---|---|---|---|---|---|---|---|
| H-6.1 | H | Me | H | Et | Cl | H | Oel, $n_D^{50} = 1.5810$ |
| H-6.2 | H | Me | H | Et | Br | H | |
| H-6.3 | H | Me | H | Me | Cl | H | |
| H-6.4 | H | Me | H | $CF_3$ | Cl | H | |
| H-6.5 | H | Me | H | $SCH_3$ | Cl | H | |
| H-6.6 | Ac | Me | H | Et | Cl | H | Smp. 52-55°C |
| H-6.7 | Ac | Me | H | Et | Br | H | |
| H-6.8 | Ac | Me | H | Me | Cl | H | |
| H-6.9 | H | Et | H | Et | Cl | H | Oel |
| H-6.10 | H | Et | H | Et | Br | H | |
| H-6.11 | H | Et | H | Me | Cl | H | |
| H-6.12 | H | Et | H | Me | Br | H | |
| H-6.13 | Ac | Et | H | Et | Cl | H | Oel |
| H-6.14 | Ac | Et | H | Et | Br | H | |
| H-6.15 | Ac | Et | H | Me | Cl | H | |
| H-6.16 | H | Me | H | Et | Cl | OMe | |
| H-6.17 | H | Me | H | Me | Cl | OMe | |
| H-6.18 | Me | Me | H | Et | Cl | H | |
| H-6.19 | Me | Et | H | Et | Cl | H | |
| H-6.20 | CO-Et | Me | H | Et | Cl | H | |
| H-6.21 | CHO | Me | H | Et | Cl | H | |
| H-6.22 | H | 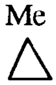 | H | Me | Cl | H | |

| Beispiel | $R_1$ | $R_2$ | $R_4$ | $R_5$ | $R_6$ | $R_{17}$' | Phys. Daten |
|---|---|---|---|---|---|---|---|
| H-6.23 | H | Me | Me | Et | Cl | H | |
| H-6.24 | $SO_2Me$ | Me | H | Et | Cl | H | |

Tabelle 7: Verbindungen der Formel I.7

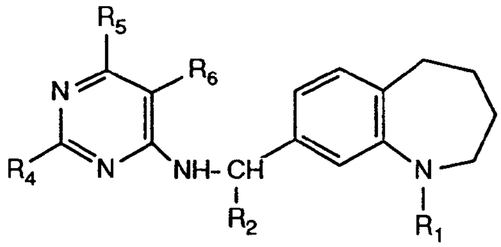

(I.7)

| Beispiel | $R_1$ | $R_2$ | $R_4$ | $R_5$ | $R_6$ | Phys. Daten |
|---|---|---|---|---|---|---|
| H-7.1 | H | Me | H | Et | Cl | Oel |
| H-7.2 | H | Me | H | Me | Cl | |
| H-7.3 | H | Me | H | Me | Br | |
| H-7.4 | H | Et | H | Et | Cl | Oel, $n_D^{50}$ = 1,5770 |
| H-7.5 | H | Et | H | Me | Cl | |
| H-7.6 | H | Et | H | Me | Br | |
| H-7.7 | Ac | | H | Et | Cl | Harz, $^1$H-NMR |
| H-7.8 | Ac | | H | Et | Cl | Oel, $n_D^{50}$ = 1,5610 |
| H-7.9 | CO-Et | | H | Et | Cl | |
| H-7.10 | CO-Et | | H | Et | Cl | |
| H-7.11 | H | | H | Et | Cl | |
| H-7.12 | Ac | | H | Et | Cl | |
| H-7.13 | Me | | H | Et | Cl | |
| H-7.14 | Me | | H | Et | Cl | |
| H-7.15 | Me | | H | Me | Cl | |
| H-7.16 | H | | Me | Et | Cl | |
| H-7.17 | H | | Me | Me | Br | |
| H-7.18 | CHO | | H | Et | Cl | |
| H-7.19 | CHO | | H | Et | Cl | |
| H-7.20 | $SO_2Me$ | | H | Et | Cl | |
| H-7.21 | $SO_2Me$ | | H | Me | Cl | |
| H-7.22 | $SO_2Ph$ | | H | Et | Cl | |

Herstellungsbeispiele für Zwischenprodukte

Beispiel H-8:

In einem Hydrierautoklaven werden 2,25 g 2-Acetylchinolin in 110 ml absolutem $CH_3OH$ gelöst. Nach Zugabe von 0,5 g Raney-Nickel und 4 g trockenem Ammoniak wird die Temperatur auf 100° erhöht und das Gemisch 30 Stunden unter einem Druck von 2.5 x $10^6$ bis 2.7 x $10^6$ Pa hydriert. Anschliessend werden nochmals 0,5 g Raney-Nickel zugegeben und das Gemisch wird weitere 10 Stunden hydriert. Nach dem Abkühlen wird der Katalysator abfiltriert und das Lösungsmittel im Wasserstrahlvakuum abgedampft. Der Rückstand wird mittels Mashchromatographie an einer kurzen Kieselgelsäule [Fliessmittel: zuerst Ethanol/Diethylether (1:1), danach Ethanol] gereinigt. Man erhält so das 2-[(1-Aminoethyl)-1,2,3,4-tetrahydro]chinolin in Form eines hellbraunen Oels, das gemäss $^1$H-NMR als Diastereoisomerengemisch (1.35 : 1) vorliegt.

Beispiel H-9:

In einem Hydrierautoklaven werden 10,7 g eines Gemisches, bestehend aus 1-Acetyl-2-methyl-6-propionyl- 1,2,3,4-tetrahydro-chinolin und 1-Acetyl-2-methyl-7-propionyl-1,2,3,4-tetrahydro-chinolin, sowie 7,9 g $NH_3$ (wasserfrei), 0,05 g Eisessig und 2,0 g Raney-Nickel in 120 ml absolutem Ethanol vorgelegt. Danach wird die Innentemperatur auf 100° erhöht und das Gemisch 7 Stunden hydriert. Danach werden nochmals 2,0 g Raney-Nickel zugegeben und das Gemisch wird weitere 12 Stunden hydriert. Nach Beendigung der Wasserstoff-Aufnahme wird abgekühlt und der Katalysator abfiltriert. Nach dem Abdampfen des Lösungsmittels, einschliesslich des überschüssigen Ammoniaks, im Wasserstrahlvakuum hinterbleibt ein Rückstand, der durch Flashchromatographie an Kieselgel (Fliessmittel: zuerst Diethylether, anschliessend Ethanol) gereinigt wird. Man erhält so ein Gemisch von 1-Acetyl-6-[(1-aminoprop-1-yl)-2-methyl-1,2,3,4-tetrahydro]chinolin und 1-Acetyl-7-[(1-aminoprop-1-yl)-2-methyl-1,2,3,4-tetrahydro]chinolin in Form eines roten Oels ($n_D^{50}$ = 1,5365;).

Beispiel H-10:

In einem Sulfierkolben werden 0,6 g Lithiumaluminiumhydrid in 30 ml absolutem Dioxan unter Stickstoff vorgelegt. Danach wird die Innentemperatur auf ca. 70°C erhöht und eine Lösung von 1,2 g 6-(1-Aminopropyl)3,4-dihydrochinolin-2-on in 30 ml absolutem Dioxan innerhalb 10 Min. zugetropft. Man rührt 6 Stunden bei einer Innentemperatur von ca. 80-85°C nach, lässt abkühlen und gibt unter Eiskühlung so lange tropfenweise Wasser zu, bis das gesamte überschüssige Lithiumaluminiumhydrid abreagiert hat. Danach werden ca. 40 ml Dioxan und Natriumsulfat (ca. 10 g) zugegeben. Nach kurzem stehen wird abgesaugt und das Dioxan im Wasserstrahlpumpenvakuum abgezogen. Das dunkelbraune, zurückbleibende Oel kann anschliessend durch Flashchromatographie an Kieselgel (Fliessmittel: Ethanol/Ether 1:1) weiter gereinigt werden. Man erhält 1,0 g 6-(1-Aminopropyl)-1,2,3,4-tetrahydrochinolin in Form eines braunen Oels ($^1$H-NMR).

Beispiel H-11:

In einer Hydrierapparatur werden 4,0 g 2-Propionylchinolin-oxim und 2 g 5 % Pd/C-Katalysator in 40 ml Eisessig vorgelegt. Es wird 5 Stunden bei 20-25°C unter Normaldruck hydriert Anschliessend wird filtriert, der Eisessig in Wasserstrahlvakuum abgezogen und der Rückstand mittels Flashchromatographie an Kieselgel (Fliessmittel: Ethanol/Ether 1:1) gereinigt. Man erhält 3,6 g 2-(1-Aminopropyl)chinolin in Form eines roten Oels ($^1$H-NMR).

Beispiel H-12:

In einem Hydrierautoklaven werden 9,2 g 6-Propionyl-3,4-dihydrochinolin-2-on-oxim, 10 g trockenes Ammoniak und 3 g Raney-Nickel in 130 ml absolutem Methanol vorgelegt. Es wird 12 Stunden bei einer Temperatur von 50°C und einem Druck von 70 bar hydriert. Danach wird der Katalysator abfiltriert und das Lösungsmittel, sowie das überschüssige $NH_3$ im Wasserstrahlvakuum abgezogen. Die Reinigung erfolgt mit Hilfe der Flashchromatographie (Fliessmittel: Ethanol/Ether 1:3) und ergibt 8,2 g 6-(1-Aminopropyl)-3,4-dihydrochinolin-2-on.

Beispiel H-13:

In einem Sulfierkolben werden 1,3 g 7-(1-Aminoethyl)-1,2,3,4-tetrahydrochinolin, 0,73 g Dimethylsulfat

und 0,27 g gemahlenes Magnesiumoxid in 50 ml absolutem Toluol vorgelegt. Danach wird die Innentemperatur auf 100-105°C erhöht und das Gemisch 18 Stunden bei dieser Temperatur gerührt. Nach dem Abkühlen werden anorganische Anteile abfiltriert und die Toluollösung mit 10 ml conc. HCl versetzt und 2 Stunden bei 70°C gehalten. Danach wird mit Natronlauge neutralisiert und die organische Phase abgetrennt. Die wässrige Phase wird anschliessend noch 2 x mit Essigester extrahiert und die organischen Phasen werden über $Na_2SO_4$ getrocknet. Nach Abziehen des Lösungsmittels im Wasserstrahlvakuum erhält man 1,2 g Rohprodukt das durch Flashchromatographie an Kieselgel (Fliessmittel: Essigester/n-Hexan 1:1) gereinigt werden kann. Man erhält 1,05 g 7-(1-Aminoethyl)-1-methyl-1,2,3,4-tetrahydrochinolin in Form eines roten Oels ($^1$H-NMR).

In analoger Weise wie in den Beispielen H-8 bis H-12 beschrieben oder mittels einer entsprechenden anderen der vorstehend angegebenen Verfahrensweisen können auch die in den nachfolgenden Tabellen aufgeführten Verbindungen hergestellt werden.

Tabelle 8: Verbindungen der Formel III.8

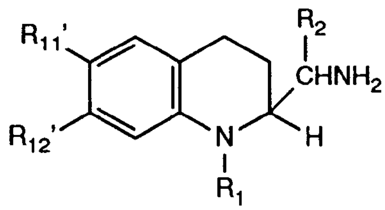

(III.8)

| Beispiel | $R_1$ | $R_2$ | $R_{11}'$ | $R_{12}'$ | Phys. Daten |
|---|---|---|---|---|---|
| H-8.1 | H | Et | H | H | Oel, $^1$H-NMR |
| H-8.2 | H | Me | OMe | H | Oel, $n_D^{50} = 1{,}5410$ |
| H-8.3 | H | Bu | H | H | |
| H-8.4 | H | Et | OMe | H | |
| H-8.5 | H | Et | H | H | |
| H-8.6 | H | Pr | OMe | H | Oel, $^1$H-NMR |
| H-8.7 | H | Me | F | H | Oel, $n_D^{50} = 1{,}5458$ |
| H-8.8 | H | Et | F | H | |
| H-8.9 | H | Me | Cl | H | Oel, $^1$H-NMR |
| H-8.10 | H | Et | Cl | H | Oel, $^1$H-NMR |
| H-8.11 | H | 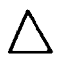 | H | H | |
| H-8.12 | H | 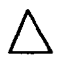 | Cl | H | |
| H-8.13 | Me | Me | H | H | |
| H-8.14 | Et | Me | H | H | |
| H-8.15 | H | Me | H | Cl | |
| H-8.16 | H | Et | H | Cl | |
| H-8.17 | H | Pr | H | H | |
| H-8.18 | H | Me | OEt | H | Oel |
| H-8.19 | H | Me | $OCF_3$ | H | |
| H-8.20 | H | Et | $OCF_3$ | H | |
| H-8.21 | H | Me | $OCHF_2$ | H | |
| H-8.22 | H | Et | $OCHF_2$ | H | |
| H-8.23 | H | Me | OPh | H | |
| H-8.24 | H | Et | OPh | H | |

| Beispiel | $R_1$ | $R_2$ | $R_{11}$' | $R_{12}$' | Phys. Daten |
|---|---|---|---|---|---|
| H-8.25 | H | Me | 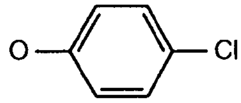 | H | Oel, MS ($M^+ = 303$) |
| H-8.26 | H | Et | 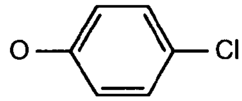 | H | Oel, $^1$H-NMR |
| H-8.27 | H | Me | 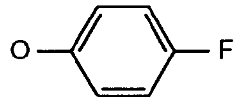 | H | |
| H-8.28 | H | Et | 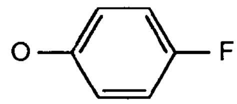 | H | |
| H-8.29 | H | Me | 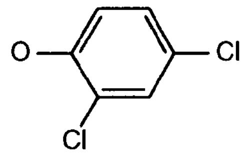 | H | |
| H-8.30 | H | Et | 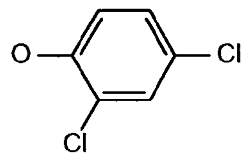 | H | |
| H-8.31 | H | Me | 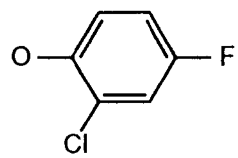 | H | |
| H-8.32 | H | Et | 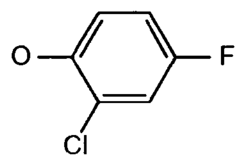 | H | |
| H-8.33 | $SO_2Me$ | Me | H | H | |
| H-8.34 | $SO_2Ph$ | Me | H | H | |

Tabelle 9: Verbindungen der Formel III.9

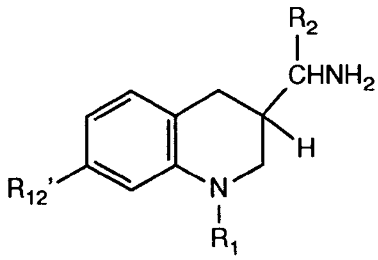

(III.9)

| Beispiel | $R_1$ | $R_2$ | $R_{12}$' | Phys. Daten |
|---|---|---|---|---|
| H-9.1 | H | Me | H | Oel, $^1$H-NMR |
| H-9.2 | H | Me | Cl | |
| H-9.3 | H | Et | H | |
| H-9.4 | H | 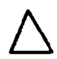 | H | |
| H-9.5 | Me | Me | H | |
| H-9.6 | Me | Et | H | |
| H-9.7 | Et | Me | H | |
| H-9.8 | Ac | Me | H | |
| H-9.9 | Ac | Et | H | |

<u>Tabelle 10:</u> Verbindungen der Formel III.10

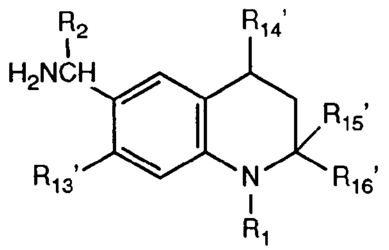

(III.10)

| Beispiel | $R_1$ | $R_2$ | $R_{13}$' | $R_{14}$' | $R_{15}$' | $R_{16}$' | Phys. Daten |
|---|---|---|---|---|---|---|---|
| H-10.1 | H | Me | H | H | H | H | Oel, $^1$H-NMR |
| H-10.2 | H | Et | Me | Me | Me | Me | Oel, $n_D^{50} = 1{,}5404$ |
| H-10.3 | H | Me | Me | Me | Me | Me | Oel |
| H-10.4 | Et | Me | H | H | H | H | |
| H-10.5 | Et | Et | H | H | H | H | |
| H-10.6 | H | Me | H | H | H | Me | |
| H-10.7 | H | Et | H | H | H | Me | |
| H-10.8 | Ac | Me | Me | Me | Me | Me | Oel, $n_D^{50} = 1{,}5295$ |
| H-10.9 | Ac | Et | Me | Me | Me | Me | Smp. 76-80°C |
| H-10.10 | H | 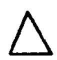 | H | H | H | H | |
| H-10.11 | Ac | Me | H | H | H | Me | |
| H-10.12 | Ac | Et | H | H | H | Me | |
| H-10.13 | Me | Me | H | H | H | H | |
| H-10.14 | Me | Et | H | H | H | H | |
| H-10.15 | Me | Me | H | H | H | Me | |
| H-10.16 | Me | Et | H | H | H | Me | |
| H-10.17 | H | 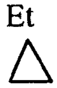 | H | H | H | Me | |

Tabelle 11: Verbindungen der Formel III.11

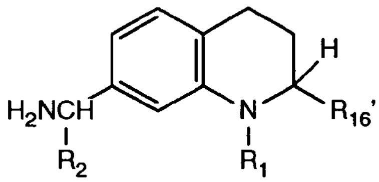

(III.11)

| Beispiel | $R_1$ | $R_2$ | $R_{16}'$ | Phys. Daten |
|---|---|---|---|---|
| H-11.1 | H | Me | H | Oel, $^1$H-NMR |
| H-11.2 | H | Et | H | Oel |
| H-11.3 | H | Me | Me | |
| H-11.4 | H | Et | Me | |
| H-11.5 | Ac | Me | H | |
| H-11.6 | Ac | Et | H | |
| H-11.7 | Me | Et | H | |
| H-11.8 | H | 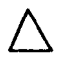 | H | |
| H-11.9 | H | 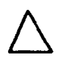 | Me | |
| H-11.10 | Et | Me | H | |
| H-11.11 | Me | Me | Me | |
| H-11.12 | Me | Et | Me | |

Tabelle 12: Verbindungsgemische der Formeln III.11 und III.12

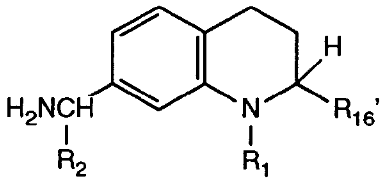 (III.11) und 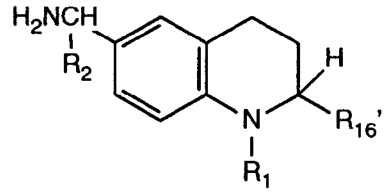 (III.12)

| Beispiel | $R_1$ | $R_2$ | $R_{16}'$ | Phys. Daten |
|---|---|---|---|---|
| H-12.1 | H | Me | H | |
| H-12.2 | Ac | Me | H | Oel, $n_D^{50} = 1{,}5342$ |
| H-12.3 | H | Et | H | Oel |
| H-12.4 | Ac | Et | H | Oel, $n_D^{50} = 1{,}5720$ |
| H-12.5 | H | Me | Me | |
| H-12.6 | H | Et | Me | |
| H-12.7 | CO-Et | Et | Me | Oel |
| H-12.8 | H | 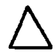 | H | |
| H-12.9 | Me | Me | H | |
| H-12.10 | Me | Et | H | |
| H-12.11 | Me | Me | Me | |
| H-12.12 | Me | Et | Me | |

<u>Tabelle 13:</u> Verbindungen der Formel III.13

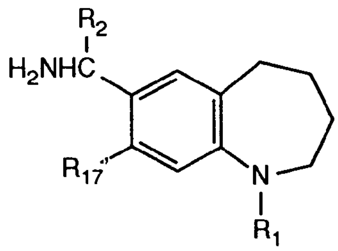

(III.13)

| Beispiel | $R_1$ | $R_2$ | $R_{17}$' | Phys. Daten |
|---|---|---|---|---|
| H-13.1 | H | Me | H | Oel, $^1$H-NMR |
| H-13.2 | Ac | Me | H | Oel, $^1$H-NMR |
| H-13.3 | H | Et | H | |
| H-13.4 | Ac | Et | H | |
| H-13.5 | H | Me | OMe | |
| H-13.6 | Me | Me | H | |
| H-13.7 | Me | Et | H | |
| H-13.8 | H | 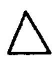 | H | |
| H-13.9 | Me | 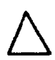 | H | |
| H-13.10 | H | 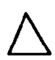 | OMe | |
| H-13.11 | CO-Et | Me | H | |
| H-13.12 | CO-Et | Et | H | |
| H-13.13 | CHO | Me | H | |
| H-13.14 | Et | Et | H | |
| H-13.15 | Et | Me | H | |

Tabelle 14: Verbindungen der Formel III.14

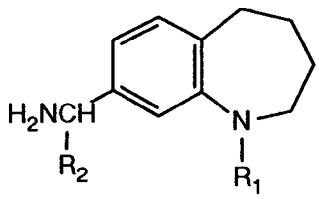

(III.14)

| Beispiel | $R_1$ | $R_2$ | Phys. Daten |
|---|---|---|---|
| H-14.1 | H | Me | Oel |
| H-14.2 | H | Et | Oel, $^1$H-NMR |
| H-14.3 | Ac | Me | Oel, $n_D^{50}$ = 1,5443 |
| H-14.4 | Ac | Et | Oel, $n_D^{50}$ = 1,5418 |
| H-14.5 | CO-Et | Me | |
| H-14.6 | CO-Et | Et | |
| H-14.7 | H | 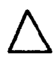 | |
| H-14.8 | Ac | 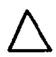 | |
| H-14.9 | Me | Me | |
| H-14.10 | Et | Me | |
| H-14.11 | Me | Et | |
| H-14.12 | Et | Et | |

Tabelle 15: Verbindungen der Formel III.15

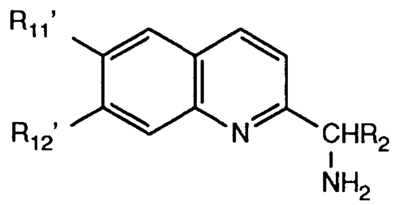

(III.15)

| Beispiel | $R_2$ | $R_{11}$' | $R_{12}$' | Phys. Daten |
|---|---|---|---|---|
| H-15.1 | Me | H | H | Oel, $n_D^{50} = 1,6084$ |
| H-15.2 | Pr | H | H | |
| H-15.3 | 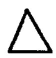 | H | H | |
| H-15.4 | Me | Cl | H | Oel, $^1$H-NMR |
| H-15.5 | Et | Cl | H | Oel, MS ($M^+ = 221$) |
| H-15.6 | 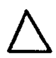 | Cl | H | |
| H-15.7 | Me | F | H | Smp. 100-104°C |
| H-15.8 | Et | F | H | |
| H-15.9 | Me | OMe | H | Smp. 64-65°C |
| H-15.10 | Et | OMe | H | |
| H-15.11 | Me | $OCF_3$ | H | |
| H-15.12 | Et | $OCF_3$ | H | |
| H-15.13 | Me | $OCHF_2$ | H | |
| H-15.14 | Et | $OCHF_2$ | H | |
| H-15.15 | Me | O-Et | H | Smp. 115-118°C |
| H-15.16 | Et | O-Et | H | |
| H-15.17 | Pr | OMe | H | Oel, $n_D^{50} = 1,5590$ |
| H-15.18 | Me | OPh | H | |
| H-15.19 | Et | OPh | H | |
| H-15.20 | Me | 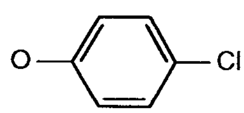 | H | Oel, $^1$H-NMR |
| H-15.21 | Et | 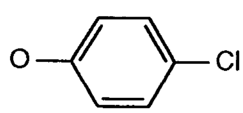 | H | Oel, $^1$H-NMR |

| Beispiel | $R_2$ | $R_{11}$' | $R_{12}$' | Phys. Daten |
|---|---|---|---|---|
| H-15.22 | Me | 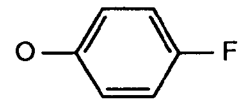 | H | |
| H-15.23 | Et | 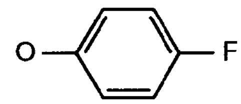 | H | |
| H-15.24 | Me | 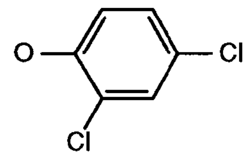 | | |
| H-15.25 | Et | 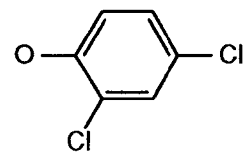 | H | |
| H-15.26 | Me | 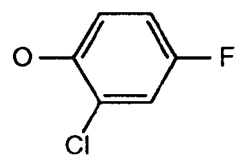 | H | |
| H-15.27 | Et | 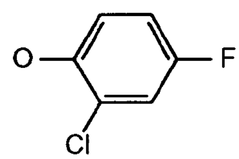 | H | |
| H-15.28 | Me | H | Cl | |
| H-15.29 | Et | H | Cl | |

Tabelle 16: Verbindungen der Formel III.16

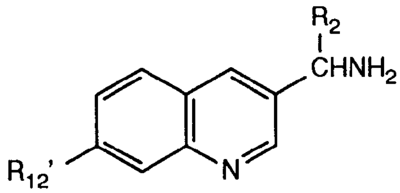

(III.16)

| Beispiel | $R_2$ | $R_{12}'$ | Phys. Daten |
|---|---|---|---|
| H-16.1 | Me | H | Oel, $^1$H-NMR |
| H-16.2 | Et | H | |
| H-16.3 | 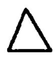 | H | |
| H-16.4 | Me | Cl | |
| H-16.5 | Et | Cl | |

Tabelle 17: Verbindungen der Formel III.17

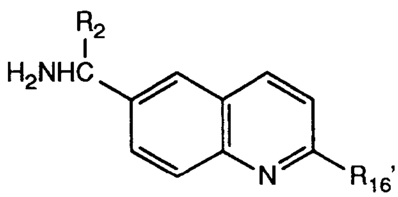

(III.17)

| Beispiel | $R_2$ | $R_{16}'$ | Phys. Daten |
|---|---|---|---|
| H-17.1 | Me | H | Oel, $^1$H-NMR |
| H-17.2 | Et | H | |
| H-17.3 | 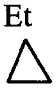 | H | |
| H-17.4 | Me | Me | |
| H-17.5 | Et | Me | |
| H-17.6 | 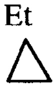 | Me | |
| H-17.7 | Pr | Me | |

Tabelle 18: Verbindungen der Formel III.18

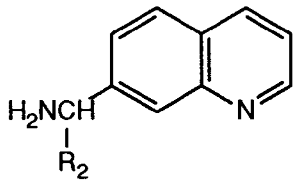

(III.18)

| Beispiel | $R_2$ | Phys. Daten |
|---|---|---|
| H-18.1 | Me | Oel, $^1$H-NMR |
| H-18.2 | Et | Oel |
| H-18.3 | Pr | |
| H-18.4 | 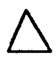 | |
| H-18.5 | Cyclopentyl | |

Tabelle 19: Verbindungen der Formel III.19

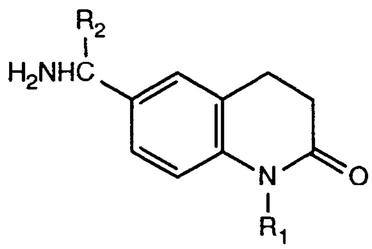

(III.19)

| Beispiel | $R_1$ | $R_2$ | Phys. Daten |
|---|---|---|---|
| H-19.1 | H | Me | Smp. 147-150°C |
| H-19.2 | H | Pr | |
| H-19.3 | H | 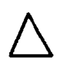 | |
| H-19.4 | Me | Me | |
| H-19.5 | Me | Et | |
| H-19.6 | Me | 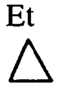 | |

Tabelle 20: Verbindungen der Formel III.20

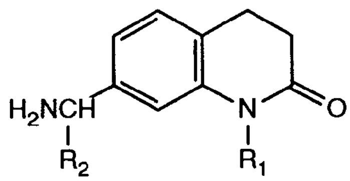

(III.20)

| Beispiel | $R_1$ | $R_2$ | Phys. Daten |
|---|---|---|---|
| H-20.1 | H | Me | |
| H-20.2 | H | Et | |
| H-20.3 | H | 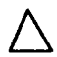 | |
| H-20.4 | Me | Me | |
| H-20.5 | Me | Et | |
| H-20.6 | Me | 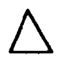 | |
| H-20.7 | Et | Me | |
| H-20.8 | Et | Et | |

Tabelle 21: Verbindungen der Formel III.21

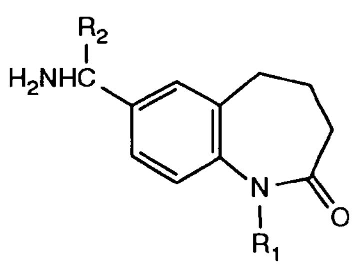

(III.21)

| Beispiel | $R_1$ | $R_2$ | Phys. Daten |
|---|---|---|---|
| H-21.1 | H | Me | Wachs, $^1$H-NMR |
| H-21.2 | H | Et | Oel, $^1$H-NMR |
| H-21.3 | H | 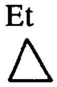 | |
| H-21.4 | Me | Me | |
| H-21.5 | Me | Et | |
| H-21.6 | Me | 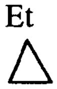 | |
| H-21.7 | Et | Me | |
| H-21.8 | Et | Et | |

Tabelle 22: Verbindungen der Formel III.22

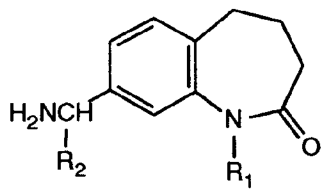

(III.22)

| Beispiel | $R_1$ | $R_2$ | Phys. Daten |
|---|---|---|---|
| H-22.1 | H | Me | |
| H-22.2 | H | Et | Oel, $^1$H-NMR |
| H-22.3 | H | 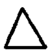 | |
| H-22.4 | Me | Me | |
| H-22.5 | Me | Et | |
| H-22.6 | Me | 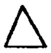 | |

Die nachfolgende Tabelle 23 enthält die $^1$H-NMR-Daten der vorgenannten Verbindungen. Die Aufnahme der $^1$H-NMR-Spektren erfolgte, sofern kein anderes Lösungsmittel angegeben ist, in $CDCl_3$. DS steht als Abkürzung für Diastereomer und Reg als Abkürzung für Regioisomer.

Tabelle 23

| Beispiel | $^1$H-NMR-Daten (ppm/Multiplizität/Anzahl der Protonen) |
|---|---|
| H-2 | 1,28/2xt/6H (Reg 1 und Reg 2); 1,57/d/6H (Reg 1 und Reg 2); 1,95/m/2H (Reg 1 und Reg 2); 2,68-2,82/m/6H (Reg 1 und Reg 2); 3,30/m/4H (Reg 1 und Reg 2); 5,23/m/2H (Reg 1 und Reg 2); 5,45-5,65/2xd/2H (Reg 1 und Reg 2); 6,49-7,02/m/6H (Reg 1 und Reg 2); 8,41/s/1H (Reg 1 oder Reg 2); 8,42/s/1H (Reg 1 oder Reg 2) |

| Beispiel | $^{1}$H-NMR-Daten (ppm/Multiplizität/Anzahl der Protonen) |
|---|---|
| H-1.22 | 1,02/t/3H; 1,28/t/3H; 1,52-1,60/m/2H; 1,78/m/1H; 1,95/m/1H; 2,80/q/2H; 3,51/m/1H; 4,40/m/1H; 4,52/s/1H; 5,15/d/1H; 6,29/d/1H; 6,86/dd/1H; 6,89/d/1H; 8,42/s/1H |
| H-1.26.1 | 1,28/d+t/6H; 1,45/m/1H; 2,07/s/3H; 2,50/m/2H; 2,75/m/1H; 2,80/q/2H; 3,89/m/1H; 4,98/q/1H; 6,65/d/1H; 6,87/m/1H; 7,11-7,21/m/3H; 8,39/s/1H |
| H-1.35.2 | 1,38/d/3H; 1,62/m/1H; 2,01/m/1H; 2,80-2,90/m/4H; 3,42/m/1H; 4,39/m/1H; 6,58/d/1H; 6,69/m/2H; 6,88/d/1H; 7,18-7,30/m/4H; 8,43/s/1H |
| H-2.12 | 1,29/t/3H; 1,32/d/3H; 2,18/m/1H; 2,68/m/1H; 2,79/q/2H; 2,90/dd/1H; 3,15/m/1H; 3,41/m/1H; 4,48/m/1H; 6,51/d/1H; 6,65/tr/1H; 6,98/m/2H; 8,40/s/1H |
| H-3.25 | 0,90/t/3H; 1,22/t/3H; 1,79-2,02/m/4H; 2,75/m/4H; 3,29/t/2H; 3,82/s/1H; 5,0/q/1H; 5,53/d/1H; 6,45/d/1H; 6,93/m/2H; 8,40/s/1H |
| H-4.18 | 1,12/t/3H; 1,25/t/3H; 1,60/o/3H; 1,94/m/2H; 2,43/q/2H; 2,70/t/2H; 2,78/q/2H; 3,75/m/1H; 5,30/q/1H; 5,59/d/1H; 7,07-7,14/m/2H; 7,27/s/1H; 8,37/s/1H |
| H-4.20 | 1,24/t/3H; 1,58/d/3H; 1,96/m/2H; 2,78/m/4H; 2,90/s/3H; 3,22/t/2H; 5,26/m/1H; 5,59/d/1H; 6,57/s/1H; 6,61/dd/1H; 6,93/o/1H; 8,41/s/1H |
| H-4.24 | 1,11/t/3H; 1,24/t/3H; 1,58/d/3H; 1,94/m/2H; 2,70-2,81/m/4H; 3,24/t/2H; 3,33/q/2H; 5,24/m/1H; 5,59/d/1H; 6,54-6,59/m/2H; 6,92/d/1H; 8,41/s/1H |
| H-4.27 | 1,12/t/3H; 1,25/t/3H; 1,60/d/3H; 1,94/m/2H; 2,43/q/2H; 2,70/t/2H; 2,78/q/2H; 3,75/m/1H; 5,30/q/1H; 5,59/d/1H; 7,07-7,14/m/2H; 7,27/s/1H; 8,37/s/1H |

| Beispiel | $^{1}$H-NMR-Daten (ppm/Multiplizität/Anzahl der Protonen) |
|---|---|
| H-5.5 | 1,20-1,35/m/6H (Reg 1 und Reg 2); 1,56-1,65/2xd/6H (Reg 1 und Reg 2); 1,88-2,02/m/4H (Reg 1 und Reg 2); 2,18/s/3H (Reg 1 oder Reg 2); 2,25/s/3H (Reg 1 oder Reg 2), 2,65-2,85/m/8H (Reg 1 und Reg 2); 3,80/t/4H (Reg 1 und Reg 2); 5,32/m/2H (Reg 1 und Reg 2); 5,59/t/2H (Reg 1 und Reg 2); 7,05-7,25/m/6H (Reg 1 und Reg 2), 8,38/s/1H (Reg 1 und Reg 2); 8,41/s/1H (Reg 1 oder Reg 2) |
| H-8 | 1,12/d/3H (DS 1 oder DS 2); 1,18/d/3H (DS 1 oder DS 2); 1,60-2,08/m/8H (DS 1 und DS 2); 2,69-2,88/m/5H (DS 1 und DS 2); 2,95/m/1H (DS 1 oder DS 2); 3,08/m/1H (DS 1 oder DS 2); 3,18/m/1H (DS 1 oder DS 2); 6,49-6,61/m/4H (DS 1 und DS 2); 6,95/m/4H (DS 1 und DS 2) |
| H-8.1 | 1,05/2xt/6H (DS 1 und DS 2); 1,20-2,05/m/8H (DS 1 und DS 2); 2,51/m/1H (DS 1 oder DS 2); 2,70-2,90/m/2H (DS 1 und DS 2); 3,05/m/1H (DS 1 oder DS 2); 3,25/m/2H (DS 1 und DS 2); 6,48-6,68/m/4H (DS 1 und DS 2); 6,90-7,0/m/4H (DS 1 und DS 2) |
| H-8.6 | 0,99/2xt/6H (DS 1 und DS 2); 1,15-2,05/m/12H (DS 1 und DS 2); 2,55-2,90/m/6H (DS 1 und DS 2); 2,98/m/1H (DS 1 oder DS 2); 3,15/m/1H (DS 1 oder DS 2); 6,48-6,62/m/6H (DS 1 und DS 2) |
| H-8.9 | 1,10/d/3H (DS 1 oder DS 2); 1,18/d/3H (DS 1 oder DS 2); 1,50-2,05/m/4H (DS 1 und DS 2); 2,65-2,93/m/5H (DS 1 und DS 2); 3,05-3,20/m/3H (DS 1 und DS 2); 6,45/m/2H (DS 1 und DS 2); 6,92/m/4H (DS 1 und DS 2) |
| H-9.1 | 1,10-1,20/2xd/6H (DS 1 und DS 2); 2,05/m/1H (DS 1 oder DS 2); 2,45-2,95/m/6H (DS 1 und DS 2); 3,10/m/1H (DS 1 oder DS 2); 3,25-3,50/m/2H (DS 1 und DS 2); 6,48/t/2H (DS 1 und DS 2); 6,60/t/2H (DS 1 und DS 2); 6,85-7,0/m/4H (DS 1 und DS 2) |

| Beispiel | $^1$H-NMR-Daten (ppm/Multiplizität/Anzahl der Protonen) |
|---|---|
| H-10 | 0,89/t/3H; 1,65/m/4H; 1,94/m/2H; 2,78/t/2H; 3,30/t/2H; 3,64/t/1H; 6,45/d/1H; 6,89/m/2H |
| H-10.1 | 1,37/d/3H; 1,95/m/2H; 2,10/s/1H; 2,79/t/2H; 3,30/m/2H; 3,98/g/1H; 6,45/m/1H; 6,95/m/2H |
| H-11 | 0,95/t/3H; 1,90/m/2H; 4,20/m/1H; 7,40/d/1H; 7,53/t/1H, 7,72/t/1H; 7,81/d/1H; 8,08/d/1H; 8,13/d/1H |
| H-11.1 | 1,39/d/3H; 1,54/m/1H; 1,91/t/2H; 2,20/m/3H; 2,75/t/2H; 3,30/t/2H; 4,0/m/1H; 6,50/s/1H; 6,58/d/1H; 6,92/d/1H |
| H-12 | 0,89/t/3H; 1,60-1,72/m/2H; 2,65/t/2H; 2,98/t/2H; 3,74/m/1H; 6,75/d/1H; 7,08-7,15/m/2H; 8,57/s/1H |
| H-13.1 | 1,39/d/3H; 1,40-2,05/m/4H; 2,76/m/2H; 3,03/t/2H; 4,03/q/1H; 6,69/d/1H; 7,00/dd/1H; 7,08/s/1H |
| H-13.2 | 1,40/d/3H; 1,70-2,05/m/4H; 1,88/s/3H; 2,55-2,81/m/3H; 4,12/q/1H; 4,69/dt/1H; 7,09/d/1H; 7,18-7,26/m/2H |
| H-14.2 | 0,89/t/3H; 1,45-1,95/m/9H; 2,75/m/2H; 3,05/m/2H; 3,71/t/1H; 6,69/d/1H; 6,76/dd/1H; 7,05/d/1H |
| H-15.4 | 1,52/d/3H; 1,60/s/2H; 4,34/q/1H; 7,49/d/1H; 7,63/dd/1H; 7,79/dd/1H; 8,0/d/1H; 8,05/d/1H |
| H-15.20 | 1,52/d/3H; 4,40/q/1H; 7,02/m/2H; 7,22/d/1H; 7,35/d/2H; 7,40-7,50/m/2H; 7,78-8,09/m/2H |
| H-15.21 | 0,97/t/3H; 1,90/m/2H; 4,18/t/1H; 7,0-7,5/m/7H; 8,0/d/1H; 8,08/d/1H |
| H-16.1 | 1,52/d/3H; 1,72/s/2H; 4,40/q/1H; 7,57/m/1H; 7,70/m/1H; 7,82/dd/1H; |

| Beispiel | $^{1}$H-NMR-Daten (ppm/Multiplizität/Anzahl der Protonen) |
|---|---|
| | 8,12/d/1H; 8,89/m/1H |
| H-17.1 | 1,50/d/3H; 4,36/q/1H; 7,40/m/1H; 7,72/dd/1H; 7,80/s/1H; 8,08/d/1H; 8,12/d/1H; 8,89/m/1H |
| H-18.1 | 1,50/d/3H; 1,70/s/2H; 4,37/q/1H; 7,39/m/1H; 7,60/dd/1H; 7,81/d/1H; 8,04/s/1H; 8,15/dd/2H; 8,90/m/1H |
| H-21.1 | 1,40/d/3H; 2,22/m/2H; 2,36/t/2H; 2,80/t/2H; 4,10/q/1H; 6,90/d/1H; 7,15-7,25/m/2H; 7,50/s/1H |
| H-21.2 | 0,90/t/3H; 1,70/t/2H; 2,25/m/2H; 2,38/m/2H; 2,80/t/2H; 3,80/m/1H; 6,90/s/1H; 7,10-7,22/m/2H; 7,29/s/1H |
| H-22.2 | 0,90/t/3H; 1,29/t/3H; 1,70/t/2H; 2,20-2,49/m/4H; 2,75/s/2H; 3,60-3,90/m/3H; 7,08/d/1H; 7,18/d/1H; 7,42/s/1H |

Beispiele F-1.1 bis F-6.3: Formulierung erfindungsgemässer Verbindungen

Beispiele F-1.1 bis F-1.3: Emulsions-Konzentrate

| Bestandteile | F-1.1 | F-1.2 | F-1.3 |
|---|---|---|---|
| Erfindungsgemässer Wirkstoff | 25% | 40% | 50% |
| Calciumdodecylbenzolsulfonat | 5% | 8% | 6% |
| Ricinusölpolyethylenglycolether (36 mol Ethylenoxyeinheiten) | 5% | - | - |
| Tributylphenolpolyethylenglycolether (30 mol Ethylenoxyeinheiten) | - | 12% | 4% |
| Cyclohexanon | - | 15% | 20% |
| Xylolgemisch | 65% | 25% | 20% |

Aus diesen Emulsions-Konzentraten können durch Verdünnung mit Wasser Emulsionen jeder gewünschten Konzentration hergestellt werden.

Beispiel F-2: Emulsions-Konzentrat

| Bestandteile | F-2 |
|---|---|
| Erfindungsgemässer Wirkstoff | 10% |
| Octylphenolpolyethylenglycolether (4 bis 5 mol Ethylnoxyeinheiten) | 3% |
| Calciumdodecylbenzolsulfonat | 3% |
| Ricinusölpolyglycolether (36 mol Ethylenoxyeinheiten) | 4% |
| Cyclohexanon | 30% |
| Xylolgemisch | 50% |

Aus diesem Emulsions-Konzentrat können durch Verdünnung mit Wasser Emulsionen jeder gewünschten Konzentration hergestellt werden.

Beispiele F-3.1 bis F-3.4: Lösungen

| Bestandteile | F-3.1 | F-3.2 | F-3.3 | F-3.4 |
|---|---|---|---|---|
| Erfindungsgemässer Wirkstoff | 80% | 10% | 5% | 95% |
| Propylenglycolmonomethylether | 20% | - | - | - |
| Polyethylenglycol (relative Molekularmasse: 400 Atommasseneinheiten) | - | 70% | - | - |
| N-Methylpyrrolid-2-on | - | 20% | - | - |
| Epoxidiertes Kokosnussöl | - | - | 1% | 5% |
| Benzin (Siedegrenzen: 160-190°) | - | - | 94% | - |

Die Lösungen sind zur Anwendung in Form kleinster Tropfen geeignet.

Beispiele F-4. 1 bis F-4.4: Granulate

| Bestandteile | F-4.1 | F-4.2 | F-4.3 | F-4.4 |
|---|---|---|---|---|
| Erfindungsgemässer Wirkstoff | 5% | 10% | 8% | 21% |
| Kaolin | 94% | - | 79% | 54% |
| Hochdisperse Kieselsäure | 1% | - | 13% | 7% |
| Attapulgit | - | 90% | - | 18% |

Der erfindungsgemässe Wirkstoff wird in Dichlormethan gelöst, die Lösung auf den Träger aufgesprüht und das Lösungsmittel anschliessend im Vakuum abgedampft.

Beispiele F-5.1 und F-5.2: Stäubemittel

| Bestandteile | F-5.1 | F-5.2 |
|---|---|---|
| Erfindungsgemässer Wirkstoff | 2% | 5% |
| Hochdisperse Kieselsäure | 1% | 5% |
| Talkum | 97% | - |
| Kaolin | - | 90% |

Durch inniges Mischen aller Bestandteile erhält man gebrauchsfertige Stäubemittel.

Beispiele F-6.1 bis F-6.3: Spritzpulver

| Bestandteile | F-6.1 | F-6.2 | F-6.3 |
|---|---|---|---|
| Erfindungsgemässer Wirkstoff | 25% | 50% | 75% |
| Natriumligninsulfonat | 5% | 5% | - |
| Natriumlaurylsulfat | 3% | - | 5% |
| Natriumdiisobutylnaphthalinsulfonat | - | 6% | 10% |
| Octylphenolpolyethylenglycolether (7 bis 8 mol Ethylenoxyeinheiten) | - | 2% | - |
| Hochdisperse Kieselsäure | 5% | 10% | 10% |
| Kaolin | 62% | 27% | - |

Alle Bestandteile werden gut gemischt und das Gemisch in einer geeigneten Mühle gut vermahlen. Man erhält Spritzpulver, die sich mit Wasser zu Suspensionen jeder gewünschten Konzentration verdünnen lassen.

Beispiele B-1.1 bis B-10: Biologische Wirkung erfindungsgemässer Verbindungen

A. Mikrobizide Wirkung

Beispiel B-1.1: Systemische Wirkung gegen Pythium ultimum auf Zuckerrüben

Testmethode: Myzel von Pythium ultimum wird mit Erde gemischt (500 ml Myzelsuspension/10l Erde) und das Gemisch in 250 ml-Plastikschalen abgefüllt. Nach viertägiger Inkubation bei 10° werden in jede Schale 10 Saatkörner der zu prüfenden Zuckerrübenpflanze gesteckt. Am nächsten Tag werden die Schalen mit je 50 ml einer einen der erfindungsgemässen Wirkstoffe enthaltenden wässrigen Spritzlösung (0,002% Aktivsubstanz) übergossen. Nach einer siebentägigen Inkubationsphase bei 10° und einer anschliessenden viertägigen Inkubationsphase bei 22° wird die Wirkung der Aktivsubstanz nach Zahl und Aussehen der aufgelaufenen Pflanzen bewertet.
Testresultat: Erfindungsgemässe Wirkstoffe zeigen gegen Pythium ultimum auf Zuckerrüben gute systemische Wirkung.

Beispiel B-1.2: Systemische Wirkung gegen Pythium ultimum auf Mais

Testmethode: Der Test wird in analoger Weise wie in Beispiel B-1.1 beschrieben durchgeführt.
Testresultat: Erfindungsgemässe Wirkstoffe zeigen gegen Pythium ultimum auf Mais gute systemische Wirkung.

Beispiel B-2: Wirkung gegen Puccinia graminis auf Weizen

a) Residual-protektive Wirkung

Testmethode: Weizenpflanzen werden 6 Tage nach Aussaat mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. Die Beurteilung der Rostpustelnentwicklung 12 Tage nach Infektion führt zur Bewertung der Wirkung der Aktivsubstanz.
Testresultat: Erfindungsgemässe Wirkstoffe zeigen gegen Puccinia graminis auf Weizen gute residual-protektive Wirkung, z. B. reduzieren die Wirkstoffe gemäss den Beispielen H-5.1 und H-7.7 den Pilzbefall auf 20 bis 5% und der Wirkstoff gemäss Beispiel H-5.21 reduziert den Pilzbefall auf 5 bis 0%. Nicht mit der Aktivsubstanz behandelte infizierte Kontrollpflanzen weisen dagegen einen Pilzbefall von 100% auf.

b) Systemische Wirkung

Testmethode: Zu Weizenpflanzen wird 5 Tage nach Aussaat eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit einer Uredosporensuspension des Pilzes infiziert. Nach einer Inkubationszeit von 48 Stunden (Bedingungen: 95 bis 100 Prozent relative Luftfeuchtigkeit bei 20°) werden die Pflanzen in einem Gewächshaus bei 22° aufgestellt. Die Beurteilung der Rostpustelnentwicklung 12 Tage nach Infektion führt zur Bewertung der Wirkung der Aktivsubstanz.
Testresultat: Erfindungsgemässe Wirkstoffe zeigen gegen Puccinia graminis auf Weizen gute systemische Wirkung.

Beispiel B-3: Wirkung gegen Phytophthora infestans auf Tomaten

a) Residual-protektive Wirkung

Testmethode: Tomatenpflanzen werden nach dreiwöchiger Anzucht mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Sporangiensuspension des Pilzes infiziert. Die Beurteilung des Pilzbefalls 5 Tage nach Infektion, während denen 90 bis 100 Prozent relative Luftfeuchtigkeit und eine Temperatur von 20° aufrechterhalten werden, führt zur Bewertung der Wirkung der Aktivsubstanz.
Testresultat: Erfindungsgemässe Wirkstoffe zeigen gegen Phytophthora infestans auf Tomaten gute residual-protektive Wirkung, z. B. reduzieren die Wirkstoffe gemäss den Beispielen H-1.1.1 und H-7.8 den Pilzbefall auf 20 bis 0% und die Wirkstoffe gemäss den Beispielen H-1.1.2, H-5.1, H-5.18, H-5.21 und H-7.7 reduzieren den Pilzbefall auf 5 bis 0%. Nicht mit der Aktivsubstanz behandelte infizierte Kontrollpflanzen weisen dagegen einen Pilzbefall von 100% auf.

b) Systemische Wirkung

Testmethode: Zu Tomatenpflanzen wird nach dreiwöchiger Anzucht eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. Nach 48 Stunden werden die Pflanzen mit einer Sporangiensuspension des Pilzes infiziert Die Beurteilung des Pilzbefalls 5 Tage nach Infektion, während denen 90 bis 100 Prozent relative Luftfeuchtigkeit und eine Temperatur von 20° aufrechterhalten werden, führt zur Bewertung der Wirkung der Aktivsubstanz.
Testresultat: Erfindungsgemässe Wirkstoffe zeigen gegen Phytophthora infestans auf Tomaten gute systemische Wirkung.

Beispiel B-4: Residual-protektive Wirkung gegen Cercospora arachidicola auf Erdnüssen

Testmethode: 10 bis 15 cm hohe Erdnusspflanzen werden mit einer aus einem einen der erfindungsgemässen Wirkstoffe enthaltenden Spritzpulver hergestellten wässrigen Spritzbrühe (0,02% Aktivsubstanz) tropfnass besprüht und 48 Stunden später mit einer Konidiensuspension des Pilzes infiziert. Die Pflanzen werden 72 Stunden bei 21° und hoher Luftfeuchtigkeit inkubiert und anschliessend bis zum Auftreten der typischen Blattflecken in einem Gewächshaus aufgestellt. Die Bewertung der Wirkung der Aktivsubstanz erfolgt 12 Tage nach Infektion aufgrund von Anzahl und Grösse der Blattflecken.
Testresultat: Erfindungsgemässe Wirkstoffe zeigen gegen Cercospora arachidicola auf Erdnüssen gute residual-protektive Wirkung, z. B. reduzieren die Wirkstoffe gemäss den Beispielen H-5.18 und H-7.8 den Pilzbefall auf 20 bis 0% und der Wirkstoff gemäss Beispiel H-7.7 reduziert den Pilzbefall auf 5 bis 0%. Nicht mit der Aktivsubstanz behandelte infizierte Kontrollpflanzen weisen dagegen einen Pilzbefall von 100% auf.

Beispiel B-5: Wirkung gegen Plasmopara viticola auf Reben

a) Residual-protektive Wirkung

Testmethode: Rebensämlinge im 4 bis 5 Blatt-Stadium werden mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Sporangiensuspension des Pilzes infiziert. Die Beurteilung des Pilzbefalls 6 Tage nach Infektion, während denen 95 bis 100 Prozent relative Luftfeuchtigkeit und eine Temperatur von 20° aufrechterhalten werden, führt zur Bewertung der Wirkung der Aktivsubstanz.
Testresultat: Erfindungsgemässe Wirkstoffe zeigen gegen Plasmopara viticola auf Reben gute präventive residual-protektive Wirkung.

b) Residual-protektive Wirkung

Testmethode: Rebensämlinge im 4 bis 5 Blatt-Stadium werden mit einer Sporangiensuspension des Pilzes infiziert, 24 Stunden in einer Feuchtkammer (Bedingungen: 95 bis 100 Prozent relative Luftfeuchtigkeit bei 20°) inkubiert und dann mit einer aus einem einen der erfindungsgemässen Wirkstoffe enthaltenden Spritzpulver hergestellten wässrigen Spritzbrühe (0,02% Aktivsubstanz) tropfnass besprüht. Nach Antrocknen des Spritzbelags werden die Pflanzen wieder in die Feuchtkammer gebracht Die Beurteilung des Pilzbefalls 6 Tage nach Infektion führt zur Bewertung der Wirkung der Aktivsubstanz.
Testresultat: Erfindungsgemässe Wirkstoffe zeigen gegen Plasmopara viticola auf Reben gute kurative residual-protektive Wirkung.

Beispiel B-6: Wirkung gegen Pyricularia oryzae auf Reis

a) Residual-protektive Wirkung

Testmethode: Reispflanzen werden nach zweiwöchiger Anzucht mit einer aus einem einen der erfindungsgemässen Wirkstoffe enthaltenden Spritzpulver hergestellten wässrigen Spritzbrühe (0,02% Aktivsubstanz) tropfnass besprüht und 48 Stunden später mit einer Konidiensuspension des Pilzes infiziert. Die Beurteilung des Pilzbefalls 5 Tage nach Infektion, während denen 95 bis 100 Prozent relative Luftfeuchtigkeit und eine Temperatur von 22° aufrechterhalten werden, führt zur Bewertung der Wirkung der Aktivsubstanz.
Testresultat: Erfindungsgemässe Wirkstoffe zeigen gegen Pyricularia oryzae auf Reis gute residual-protektive Wirkung.

b) Systemische Wirkung

Testmethode: Zu 2 Wochen alten Reispflanzen wird eine aus einem einen der erfindungsgemässen Wirkstoffe enthaltenden Spritzpulver hergestellte wässrige Spritzbrühe (0,006% Aktivsubstanz, bezogen auf das Bodenvolumen) gegossen. Es wird dabei darauf geachtet, dass die Spritzbrühe nicht mit oberirdischen Pflanzenteilen in Berührung kommt. Dann werden die Töpfe so weit mit Wasser gefüllt, dass die untersten Stengelteile der Reispflanzen im Wasser stehen. Nach 96 Stunden werden die Pflanzen mit einer Konidiensuspension des Pilzes infiziert. Die Beurteilung des Pilzbefalls 5 Tage nach Infektion, während denen 95 bis 100 Prozent relative

Luftfeuchtigkeit und eine Temperatur von 24° aufrechterhalten werden, führt zur Bewertung der Wirkung der Aktivsubstanz.
Testresultat: Erfindungsgemässe Wirkstoffe zeigen gegen Pyricularia oryzae auf Reis gute systemische Wirkung.

Beispiel B-7: Residual-protektive Wirkung gegen Venturia inaequalis auf Aepfeln

Testmethode: Apfelstecklinge mit 10 bis 20 cm langen Frischtrieben werden mit einer aus einem einen der erfindungsgemässen Wirkstoffe enthaltenden Spritzpulver hergestellten wässrigen Spritzbrühe (0,02% Aktivsubstanz) tropfnass besprüht und 24 Stunden später mit einer Konidiensuspension des Pilzes infiziert. Die Pflanzen werden 5 Tage bei 90 bis 100 Prozent relativer Luftfeuchtigkeit inkubiert und weitere 10 Tage in einem Gewächshaus bei 20 bis 24° aufgestellt. Die Beurteilung des Schorfbefalls 15 Tage nach Infektion führt zur Bewertung der Wirkung der Aktivsubstanz.
Testresultat: Erfindungsgemässe Wirkstoffe zeigen gegen Venturia inaequalis auf Aepfeln gute residual-protektive Wirkung.

Beispiel B-8: Wirkung gegen Erysiphe graminis auf Gerste

a) Residual-protektive Wirkung

Testmethode: Ungefähr 8 cm hohe Gerstenpflanzen werden mit einer aus einem einen der erfindungsgemässen Wirkstoffe enthaltenden Spritzpulver hergestellten wässrigen Spritzbrühe (0,02% Aktivsubstanz) tropfnass besprüht und 3 bis 4 Stunden später mit Konidien des Pilzes bestäubt Die infizierten Pflanzen werden in einem Gewächshaus bei 22° aufgestellt. Die Beurteilung des Pilzbefalls 10 Tage nach Infektion führt zur Bewertung der Wirkung der Aktivsubstanz.
Testresultat: Erfindungsgemässe Wirkstoffe zeigen gegen Erysiphe graminis auf Gerste gute residual-protektive Wirkung, z. B. reduzieren die Wirkstoffe gemäss den Beispielen H-1.1.1, H-1.8 und H-5.1 den Pilzbefall auf 20 bis 0% und die Wirkstoffe gemäss den Beispielen H-1.1.2, H-5.5 und H-5.21 reduzieren den Pilzbefall auf 5 bis 0%. Nicht mit der Aktivsubstanz behandelte infizierte Kontrollpflanzen weisen dagegen einen Pilzbefall von 100% auf.

b) Systemische Wirkung

Testmethode: Zu ungefähr 8 cm hohen Gerstenpflanzen wird eine aus einem einen der erfindungsgemässen Wirkstoffe enthaltenden Spritzpulver hergestellte wässrige Spritzbrühe (0,002% Aktivsubstanz, bezogen auf das Bodenvolumen) gegossen. Es wird dabei darauf geachtet, dass die Spritzbrühe nicht mit oberirdischen Pflanzenteilen in Berührung kommt. 48 Stunden später werden die Pflanzen mit Konidien des Pilzes bestäubt. Die infizierten Pflanzen werden in einem Gewächshaus bei 22° aufgestellt. Die Beurteilung des Pilzbefalls 10 Tage nach Infektion führt zur Bewertung der Wirkung der Aktivsubstanz.
Testresultat: Erfindungsgemässe Wirkstoffe zeigen gegen Erysiphe graminis auf Gerste gute systemische Wirkung.

Beispiel B-9: Wirkung gegen Podosphaera leucotricha auf Apfeltrieben Residual-protektive Wirkung

Apfelstecklinge mit ca. 15 cm langen Frischtrieben werden mit einer Spritzbrühe (0,06 % Aktivsubstanz) besprüht. Nach 24 Stunden werden die behandelten Pflanzen mit einer Konidiensuspension des Pilzes infiziert und in einer Klimakammer bei 70 % relativer Luftfeuchtigkeit und 20°C aufgestellt. Die Beurteilung des Pilzbefalls erfolgt 12 Tage nach der Infektion.
Testresultat: Erfindungsgemässe Wirkstoffe zeigen gegen Podosphaera auf Apfeltrieben gute Wirkung.

Beispiel B-10: Wirkung gegen Tetranychus urticae auf Bohnen

Testmethode: Junge Bohnenpflanzen werden mit einer Mischpopulation von Tetranychus urticae besiedelt, einen Tag später mit einer aus einem einen der erfindungsgemässen Wirkstoffe enthaltenden Spritzpulver hergestellten wässrigen Emulsions-Spritzbrühe (0,04% Aktivsubstanz) tropfnass besprüht und anschliessend 6 Tage bei 25° inkubiert. Die Wirkung der Aktivsubstanz wird sodann auf der Basis einer Auszählung der Schädlinge bewertet. Dabei werden jeweils die toten Eier, toten Larven und toten Adulte an den behandelten Pflanzen gezählt und in analoger Weise die entsprechenden Zahlenwerte an nicht mit der Aktivsubstanz behandelten

Kontrollpflanzen ermittelt. Aus den jeweiligen Wertepaaren für die behandelten und die unbehandelten Pflanzen wird der Prozentsatz berechnet, um welchen die Schädlingspopulation an den behandelten Pflanzen reduziert worden ist (Prozent Wirkung der Aktivsubstanz).
Testresultat: Erfindungsgemässe Wirkstoffe zeigen gegen Tetranychus urticae auf Bohnen gute Wirkung.

B. Akarizide/Insektizide Wirkung

Beispiel B-11: Wirkung gegen Tetranychus cinnabarinus auf Buschbohnen

Testmethode (Verdünnungsreihe): Buschbohnen im 2 Blatt-Stadium werden mit einer Mischpopulation (Eier, Larven/Nymphen und Adulte) eines OP-toleranten Stammes von Tetranychus cinnabarinus besiedelt. Der erfindungsgemässe Wirkstoff wird 24 Stunden später in Konzentrationen von 200, 100 und 50 mg/ml in der automatischen Spritzkabine auf die Pflanzen appliziert (der Wirkstoff ist formuliert und wird jeweils mit Wasser bis zu der entsprechenden Konzentration verdünnt). Die Wirkung der Aktivsubstanz wird 2 und 7 Tage nach der Applikation auf der Basis einer Auszählung der Schädlinge bewertet. Dabei werden jeweils die toten Eier, toten Larven/Nymphen und toten Adulte an den (mit Wirkstoff in der jeweiligen Konzentration) behandelten Pflanzen gezählt und in analoger Weise die entsprechenden Zahlenwerte an nicht mit der Aktivsubstanz behandelten Kontrollpflanzen ermittelt. Aus den jeweiligen Wertepaaren für die (mit Wirkstoff in der jeweiligen Konzentration) behandelten und die unbehandelten Pflanzen wird der Prozentsatz berechnet, um welchen die Schädlingspopulation an den behandelten Pflanzen bei der jeweiligen Wirkstoffkonzentration reduziert worden ist (Prozent Wirkung der Aktivsubstanz).
Testresultat: Erfindungsgemässe Wirkstoffe zeigen gegen Tetranychus cinnabarinus auf Buschbohnen gute Wirkung.

B-12: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Testresultat: Erfindungsgemässe Wirkstoffe zeigen gegen Nilaparvata lugens gute Wirkung.

B-13: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (prozentuale Reduktion der Population).
Testresultat: Erfindungsgemässe Wirkstoffe zeigen gegen Heliothis virescens gute Wirkung.

B-14: Wirkung gegen Plutella xylostella Raupen

Junge Kohlpflanzen werden mit einer wässrigen Emulsions- Spritzbrühe, die 400 ppm des Wirkstoffes enthält, besprüht. Nach dem Antrocknen des Spritzbelages werden die Kohlpflanzen mit 10 Raupen des dritten Stadiums von Plutella xylostella besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.
Testresultat: Erfindungsgemässe Wirkstoffe, insbesondere die Verbindungen H-1.1.1 und H-1.1.2 zeigen eine gute Wirkung gegen Plutella xylostella.

**Patentansprüche**

1. Eine Verbindung der Formel

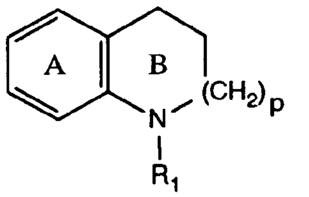

(I),

worin eines der (p + 6) substituierbaren Ring-Kohlenstoffatome der beiden Ringe A und B durch eine Gruppe der Formel Ia

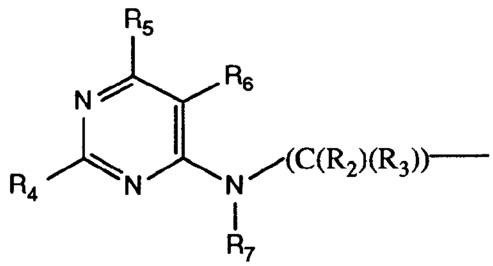

(Ia)

substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_8$-Alkyl, Halogen-$C_1$-$C_8$-alkyl, $C_1$-$C_4$-Alkoxy-$C_1$-$C_4$-alkyl, $C_3$-$C_8$-Cycloalkyl, $C_1$-$C_8$-Alkoxy, Halogen-$C_1$-$C_8$-alkoxy, $C_3$-$C_8$-Cycloalkoxy, $C_1$-$C_8$-Alkylthio, Halogen-$C_1$-$C_8$-alkylthio, Cyano, Nitro, Phenyl, Phenoxy und Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_4$-Alkyl und $C_1$-$C_4$-Alkoxy, substituiert sind, mit der Massgabe, dass höchstens einer der gegebenenfalls vorhandenen Substituenten an den erwähnten (p + 5) substituierbaren Ring-Kohlenstoffatomen der beiden Ringe A und B gegebenenfalls wie vorstehend erwähnt substituiertes Phenyl, gegebenenfalls wie vorstehend erwähnt substituiertes Phenoxy oder gegebenenfalls wie vorstehend erwähnt substituiertes Phenylthio ist, besteht, ausgewählt sind;

wobei bedeuten:

$R_1$ Wasserstoff, $C_1$-$C_8$-Alkyl, $C_3$-$C_8$-Cycloalkyl, Benzyl, $C_1$-$C_8$-Alkanoyl, das unsubstituiert oder durch Halogen oder $C_1$-$C_4$-Alkoxy substituiert ist, Benzoyl, dessen Phenylgruppe unsubstituiert oder durch einen oder zwei Substituenten, ausgewählt aus der Gruppe bestehend aus Halogen, $C_1$-$C_4$-Alkyl, Nitro und Cyano, substituiert ist; $C_1$-$C_8$-Alkansulfonyl, Halogen-$C_1$-$C_8$-alkansulfonyl, Cyano-$C_1$-$C_8$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_4$-Alkyl, Nitro und Cyano, substituiert ist;

$R_2$, $R_3$, unabhängig voneinander, Wasserstoff, $C_1$-$C_8$-Alkyl, Halogen-$C_1$-$C_8$-alkyl, $C_1$-$C_4$-Alkoxy-$C_1$-$C_4$-alkyl oder $C_3$-$C_8$-Cycloalkyl;

$R_4$ Wasserstoff, $C_1$-$C_8$-Alkyl, $C_1$-$C_8$-Alkoxy oder $C_1$-$C_8$-Alkylthio;

$R_5$ Wasserstoff, $C_1$-$C_8$-Alkyl, Halogen-$C_1$-$C_8$-alkyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkansulfinyl-$C_1$-$C_4$-alkyl, $C_1$-$C_4$-Alkansulfonyl-$C_1$-$C_4$-alkyl, $C_2$-$C_8$-Alkenyl, Halogen-$C_2$-$C_8$-alkenyl, $C_2$-$C_8$-Alkinyl, $C_3$-$C_8$-Cycloalkyl, $C_1$-$C_8$-Alkylthio oder Halogen;

$R_6$ Wasserstoff, Hydroxy, $C_1$-$C_8$-Alkyl, Halogen-$C_1$-$C_8$-alkyl, $C_1$-$C_4$-Alkoxy-$C_1$-$C_4$-alkyl, $C_1$-$C_8$-Alkoxy, $C_1$-$C_8$-Alkylthio, $C_1$-$C_8$-Alkansulfinyl, $C_1$-$C_8$-Alkansulfonyl, Halogen, Nitro, Cyano, Amino, eine Gruppe der Formel $N(H)R_8$ (Ib), eine Gruppe der Formel $N(R_8)R_9$ (Ic) oder eine Gruppe der Formel $N{=}C(R_9)R_{10}$ (Id);

$R_7$ Wasserstoff, $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_4$-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind;

$R_8$ $C_1$-$C_8$-Alkyl, Benzyl, $C_1$-$C_8$-Alkanoyl, Phenylcarbonyl, dessen Phenylgruppe unsubstituiert oder durch einen, zwei oder drei Substituenten, ausgewählt aus der Gruppe bestehend aus Halogen und C1-C4-Alkyl, substituiert ist, $C_1$-$C_8$-Alkylthio, Halogen-$C_1$-$C_8$-alkylthio, Cyano-$C_1$-$C_8$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_4$-Alkyl, Nitro und Cyano, substituiert sind;
$R_9$ $C_1$-$C_8$-Alkyl;
$R_{10}$ Wasserstoff oder $C_1$-$C_8$-Alkyl;
p 1 oder 2;
oder gegebenenfalls ein Tautomeres davon, jeweils in freier Form oder in Salzform.

2. Eine Verbindung gemäss Anspruch 1 der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_3$-$C_6$-Cycloalkyl, $C_1$-$C_4$-Alkoxy, Halogen-$C_1$-$C_4$-alkoxy mit 1, 2 oder 3 Halogenatomen, $C_3$-$C_7$-Cycloalkoxy, $C_1$-$C_4$-Alkylthio, Halogen-$C_1$-$C_4$-alkylthio mit 1, 2 oder 3 Halogenatomen, Cyano, Nitro, Phenyl, Phenoxy und Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_2$-Alkyl und $C_1$-$C_2$-Alkoxy, substituiert sind, mit der Massgabe, dass höchstens einer der gegebenenfalls vorhandenen Substituenten an den erwähnten (p + 5) substituierbaren Ring-Kohlenstoffatomen der beiden Ringe A und B gegebenenfalls wie vorstehend erwähnt substituiertes Phenyl, gegebenenfalls wie vorstehend erwähnt substituiertes Phenoxy oder gegebenenfalls wie vorstehend erwähnt substituiertes Phenylthio ist, besteht, ausgewählt sind;
wobei bedeuten:
$R_1$ Wasserstoff, $C_1$-$C_4$-Alkyl, $C_3$-$C_7$-Cycloalkyl, Benzyl, $C_1$-$C_4$-Alkanoyl, das unsubstituiert oder durch Halogen oder $C_1$-$C_2$-Alkoxy substituiert ist, Benzoyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist; $C_1$-$C_4$-Alkansulfonyl, Halogen-$C_1$-$C_4$-alkansulfonyl mit 1, 2 oder 3 Halogenatomen, Cyano-$C_1$-$C_4$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;
$R_2$, $R_3$, unabhängig voneinander, Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl oder $C_3$-$C_7$-Cycloalkyl;
$R_4$ Wasserstoff, $C_1$-$C_4$-Alkyl, $C_1$-$C_4$-Alkoxy oder $C_1$-$C_4$-Alkylthio;
$R_5$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$-$C_4$-alkyl, $C_1$-$C_2$-Alkansulfonyl-$C_1$-$C_4$-alkyl, $C_2$-$C_4$-Alkenyl, Halogen-$C_2$-$C_4$-alkenyl mit 1, 2 oder 3 Halogenatomen, $C_2$-$C_4$-Alkinyl, $C_3$-$C_7$-Cycloalkyl, $C_1$-$C_4$-Alkylthio oder Halogen;
$R_6$ Wasserstoff, Hydroxy, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro, Cyano, Amino, eine Gruppe der Formel $N(H)R_8$ (Ib), eine Gruppe der Formel $N(R_8)R_9$ (Ic) oder eine Gruppe der Formel $N{=}C(R_9)R_{10}$(Id);
$R_7$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_8$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-

$C_2$-Alkyl, Nitro und Cyano, substituiert sind;
$R_9$ $C_1$-$C_4$-Alkyl;
$R_{10}$ Wasserstoff oder $C_1$-$C_4$-Alkyl;
p 1 oder 2;
oder gegebenenfalls ein Tautomeres davon, jeweils in freier Form oder in Salzform.

3. Eine Verbindung gemäss Anspruch 1 der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass, wenn p 1 ist, die Gruppe Ia in 2-, 3- 6- oder 7-Position des in der Formel I dargestellten, aus den beiden Ringen A und B gebildeten, bicyclischen Ringsystems gebunden ist, mit der weiteren Massgabe, dass, wenn p 2 ist, die Gruppe Ia in 2-, 3-, 7- oder 8-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_3$-$C_6$-Cycloalkyl, $C_1$-$C_4$-Alkoxy, Halogen-$C_1$-$C_4$-alkoxy mit 1, 2 oder 3 Halogenatomen, $C_3$-$C_7$-Cycloalkoxy, $C_1$-$C_4$-Alkylthio, Halogen-$C_1$-$C_4$-alkylthio mit 1, 2 oder 3 Halogenatomen, Cyano, Nitro, Phenyl, Phenoxy und Phenylthio, wobei die Phenylgruppen in Phenyl, Phenoxy und Phenylthio unsubstituiert oder durch einen, zwei oder drei Substituenten, ausgewählt aus der Gruppe bestehend aus Halogen, $C_1$-$C_2$-Alkyl und $C_1$-$C_2$-Alkoxy, substituiert sind, mit der Massgabe, dass höchstens einer der gegebenenfalls vorhandenen Substituenten an den erwähnten (p + 5) substituierbaren Ring-Kohlenstoffatomen der beiden Ringe A und B gegebenenfalls wie vorstehend erwähnt substituiertes Phenyl, gegebenenfalls wie vorstehend erwähnt substituiertes Phenoxy oder gegebenenfalls wie vorstehend erwähnt substituiertes Phenylthio ist, besteht, ausgewählt sind;
wobei bedeuten:
$R_1$ Wasserstoff, $C_1$-$C_4$-Alkyl, $C_3$-$C_7$-Cycloalkyl, Benzyl, $C_1$-$C_4$-Alkanoyl, das unsubstituiert oder durch Halogen oder $C_1$-$C_2$-Alkoxy substituiert ist, Benzoyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist; $C_1$-$C_4$-Alkansulfonyl, Halogen-$C_1$-$C_4$-alkansulfonyl mit 1, 2 oder 3 Halogenatomen, Cyano-$C_1$-$C_4$-alkansulfonyl oder Phenylsulfonyl, dessen Phenylgruppe unsubstituiert oder durch einen oder zwei Substituenten, ausgewählt aus der Gruppe bestehend aus Halogen, $C_1$-$C_2$-Alkyl, Nitro und Cyano, substituiert ist;
$R_2$, $R_3$, unabhängig voneinander, Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl oder $C_3$-$C_7$-Cycloalkyl;
$R_4$ Wasserstoff, $C_1$-$C_4$-Alkyl, $C_1$-$C_4$-Alkoxy oder $C_1$-$C_4$-Alkylthio;
$R_5$ Wasserstoff, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_2$-Alkylthio-$C_1$-$C_4$-alkyl, $C_1$-$C_2$-Alkansulfinyl-$C_1$-$C_4$-alkyl, $C_1$-$C_2$-Alkansulfonyl-$C_1$-$C_4$-alkyl, $C_2$-$C_4$-Alkenyl, Halogen-$C_2$-$C_4$-alkenyl mit 1, 2 oder 3 Halogenatomen, $C_2$-$C_4$-Alkinyl, $C_3$-$C_7$-Cycloalkyl, $C_1$-$C_4$-Alkylthio oder Halogen;
$R_6$ Wasserstoff, Hydroxy, $C_1$-$C_4$-Alkyl, Halogen-$C_1$-$C_4$-alkyl mit 1, 2 oder 3 Halogenatomen, $C_1$-$C_2$-Alkoxy-$C_1$-$C_4$-alkyl, $C_1$-$C_4$-Alkoxy, $C_1$-$C_4$-Alkylthio, $C_1$-$C_4$-Alkansulfinyl, $C_1$-$C_4$-Alkansulfonyl, Halogen, Nitro, Cyano, Amino, eine Gruppe der Formel $N(H)R_8$ (Ib), eine Gruppe der Formel $N(R_8)R_9$ (Ic) oder eine Gruppe der Formel $N{=}C(R_9)R_{10}$ (Id);
$R_7$ Wasserstoff, $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;
$R_8$ $C_1$-$C_4$-Alkyl, Benzyl, $C_1$-$C_4$-Alkanoyl, Phenylcarbonyl, dessen Phenylgruppe unsubstituiert oder durch einen, zwei oder drei Substituenten, ausgewählt aus der Gruppe bestehend aus Halogen und $C_1$-$C_2$-Alkyl, substituiert ist, $C_1$-$C_4$-Alkylthio, Halogen-$C_1$-$C_4$-alkylthio mit 1, 2 oder 3 Halogenatomen, Cyano-$C_1$-$C_4$-alkylthio, Phenylthio oder Benzylthio, wobei die Phenylgruppen in Phenylthio und Benzylthio unsubstituiert oder durch einen oder zwei Substituenten, ausgewählt aus der Gruppe bestehend aus Halogen, $C_1$-$C_2$-Alkyl, Nitro und Cyano, substituiert sind;

$R_9$ $C_1$-$C_4$-Alkyl;
$R_{10}$ Wasserstoff oder $C_1$-$C_4$-Alkyl;
p 1 oder 2;
oder gegebenenfalls ein Tautomeres davon, jeweils in freier Form oder in Salzform.

4. Eine Verbindung gemäss Anspruch 1 der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass, wenn p 1 ist, die Gruppe Ia in 2-, 3-, 6- oder 7-Position des in der Formel I dargestellten, aus den beiden Ringen A und B gebildeten, bicyclischen Ringsystems gebunden ist, mit der weiteren Massgabe, dass, wenn p 2 ist, die Gruppe Ia in 2-, 3-, 7- oder 8-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl und $C_1$-$C_4$-Alkoxy besteht, ausgewählt sind;
wobei bedeuten:
$R_1$ Wasserstoff, $C_1$-$C_4$-Alkyl oder $C_1$-$C_4$-Alkanoyl;
$R_2$, $R_3$, unabhängig voneinander, Wasserstoff oder $C_1$-$C_4$-Alkyl;
$R_4$ Wasserstoff oder $C_1$-$C_4$-Alkyl;
$R_5$ $C_1$-$C_4$-Alkyl, Halogen-$C_1$-$C_4$-alkyl mit 1, 2 oder 3 Halogenatomen, $C_2$-$C_4$-Alkinyl, $C_3$-$C_4$-Cycloalkyl oder $C_1$-$C_4$-Alkylthio;
$R_6$ $C_1$-$C_4$-Alkylthio, Halogen, Nitro oder Amino;
$R_7$ Wasserstoff oder $C_1$-$C_4$-Alkyl;
p 1 oder 2;
oder gegebenenfalls ein Tautomeres davon, jeweils in freier Form oder in Salzform.

5. Eine Verbindung gemäss Anspruch 1 der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass, wenn p 1 ist, die Gruppe Ia in 2-, 3-, 6- oder 7-Position des in der Formel I dargestellten, aus den beiden Ringen A und B gebildeten, bicyclischen Ringsystems gebunden ist, mit der weiteren Massgabe, dass, wenn p 2 ist, die Gruppe Ia in 2-, 3-, 7- oder 8-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl und $C_1$-$C_4$-Alkoxy besteht, ausgewählt sind;
wobei bedeuten:
$R_1$ Wasserstoff, $C_1$-$C_4$-Alkyl oder $C_1$-$C_4$-Alkanoyl;
$R_2$, $R_3$, unabhängig voneinander, Wasserstoff oder $C_1$-$C_4$-Alkyl;
$R_4$ Wasserstoff;
$R_5$ $C_1$-$C_4$-Alkyl;
$R_6$ Halogen;
$R_7$ Wasserstoff;
p 1 oder 2;
in freier Form oder in Salzform.

6. Eine Verbindung gemäss Anspruch 1 der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass die Gruppe Ia in 2-, 3-, 6- oder 7-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus Halogen, $C_1$-$C_4$-Alkyl und $C_1$-$C_4$-Alkoxy besteht, ausgewählt sind;
wobei bedeuten:
$R_1$ Wasserstoff oder $C_1$-$C_4$-Alkanoyl;
$R_2$ Wasserstoff;
$R_3$ $C_1$-$C_4$-Alkyl;
$R_4$ Wasserstoff;
$R_5$ $C_1$-$C_4$-Alkyl;
$R_6$ Halogen;
$R_7$ Wasserstoff;
p 1;
in freier Form oder in Salzform.

7. Eine Verbindung gemäss Anspruch 1 der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder ein oder, unabhängig voneinander, zwei oder drei dieser anderen (p + 5) substituierbaren Ring-Kohlenstoffatome der beiden Ringe A und B einfach oder im Falle der gesättigten Ring-Kohlenstoffatome des Ringes B einfach oder, unabhängig voneinander, zweifach substituiert sind, mit der Massgabe, dass die Gruppe Ia in 6- oder 7-Position des in der Formel I dargestellten, aus den beiden Ringen A und B gebildeten, bicyclischen Ringsystems gebunden ist, wobei die gegebenenfalls vorhandenen Substituenten an den erwähnten (p + 5) substituierbaren Ring-Kohlenstoffatomen der beiden Ringe A und B aus der Gruppe, die aus $C_1$-$C_2$-Alkyl besteht, ausgewählt sind;
wobei bedeuten:
$R_1$ Wasserstoff oder $C_1$-$C_2$-Alkanoyl;
$R_2$ Wasserstoff;
$R_3$ $C_1$-$C_2$-Alkyl;
$R_4$ Wasserstoff;
$R_5$ $C_1$-$C_2$-Alkyl;
$R_6$ Chlor;
$R_7$ Wasserstoff;
p 1;
in freier Form oder in Salzform.

8. Eine Verbindung gemäss Anspruch 1 der Formel I, worin eines der (p + 6) substituierbaren Ring-Kohlenstoffatome der beiden Ringe A und B durch eine Gruppe Ia substituiert ist und die anderen (p + 5) substituierbaren Ring-Kohlenstoffatome der beiden Ringe A und B unsubstituiert sind oder eines dieser anderen (p + 5) substituierbaren Ring-Kohlenstoffatome der beiden Ringe A und B, insbesondere das Ring-Kohlenstoffatom in 2-Position des in der Formel I dargestellten, aus den beiden Ringen A und B gebildeten, bicyclischen Ringsystems, einfach durch $C_1$-$C_2$-Alkyl substituiert ist, mit der Massgabe, dass die Gruppe Ia in 7-Position des in der Formel I dargestellten, aus den beiden Ringen A und B gebildeten, bicyclischen Ringsystems gebunden ist;
wobei bedeuten:
$R_1$ Wasserstoff oder $C_1$-$C_2$-Alkanoyl;
$R_2$ Wasserstoff;
$R_3$ $C_1$-$C_2$-Alkyl;
$R_4$ Wasserstoff;
$R_5$ $C_1$-$C_2$-Alkyl;
$R_6$ Chlor;
$R_7$ Wasserstoff;
p 1;
in freier Form oder in Salzform.

9. Eine Verbindung gemäss Anspruch 1 der Formel I, ausgewählt aus der Gruppe von Verbindungen bestehend aus
(a) 2-[1-(5-Chlor-6-ethyl-pyrimidin-4-ylamino)prop-1-yl]-1,2,3,4-tetrahydro-chinolin,
(b) 2-[1-(5-Chlor-6-ethyl-pyrimidin-4-ylamino)ethyl]-1,2,3,4-tetrahydro-chinolin (Diastereomer 1),
(c) 2-[1-(5-Chlor-6-ethyl-pyrimidin-4-ylamino)ethyl]-1,2,3,4-tetrahydro-chinolin (Diastereomer 2),

(d) 2-[1-(5-Chlor-6-ethyl-pyrimidin-4-ylamino)ethyl]-6-methoxy-1,2,3,4-tetrahydro-chinolin,
(e) 6-[1-(5-Chlor-6-ethyl-pyrimidin-4-ylamino)ethyl]-1,2,3,4-tetrahydro-chinolin,
(f) 7-[1-(5-Chlor-6-ethyl-pyrimidin-4-ylamino)ethyl]-1,2,3,4-tetrahydro-chinolin,
(g) dem Gemisch aus 6-[1-(5-Chlor-6-ethyl-pyrimidin-4-ylamino)ethyl]-1,2,3,4-tetrahydro-chinolin und 7-[1-(5-Chlor-6-ethyl-pyrimidin-4-ylamino)ethyl]-1,2,3,4-tetrahydro-chinolin,
(h) dem Gemisch aus 1-Acetyl-6-[1-(5-chlor-6-ethyl-pyrimidin-4-ylamino)ethyl]-1,2,3,4-tetrahydro-chinolin und 1-Acetyl-7-[1-(5-chlor-6-ethyl-pyrimidin-4-ylamino)-ethyl] - 1,2,3,4-tetrahydro-chinolin,
(i) dem Gemisch aus 1-Acetyl-6-[1-(5-chlor-6-ethyl-pyrimidin-4-ylamino)prop-1-yl]-2-methyl- 1,2,3,4-tetrahydro-chinolin und 1-Acetyl-7-[1-(5-chlor-6-ethyl-pyrimidin-4-ylamino)prop-1-yl]-2-methyl-1,2,3,4-tetrahydro-chinolin,
(j) dem Gemisch aus 6-[1-(5-Chlor-6-ethyl-pyrimidin-4-ylamino)prop-1-yl]-2-methyl-1,2,3,4-tetrahydro-chinolin und 7-[1-(5-Chlor-6-ethyl-pyrimidin-4-ylamino)prop-1-yl]-2-methyl- 1,2,3,4-tetrahydro-chinolin,
(k) 1-Acetyl-8-[1-(5-chlor-6-ethyl-pyrimidin-4-ylamino)ethyl]-2,3,4,5-tetrahydro-1H-benzo[b]azepin und
(l) 1-Acetyl-8-[1-(5-chlor-6-ethyl-pyrimidin-4-ylamino)prop-1-yl]-2,3,4,5-tetrahydro-1H-benzo[b]azepin,

jeweils in freier Form oder in Salzform.

**10.** Verfahren zur Herstellung einer Verbindung gemäss Anspruch 1 der Formel I oder gegebenenfalls eines Tautomeren davon, jeweils in freier Form oder in Salzform, dadurch gekennzeichnet, dass man

a) zur Herstellung einer Verbindung der Formel I, worin $R_7$ Wasserstoff, $C_1$-$C_8$-Alkyl oder Benzyl ist, oder gegebenenfalls eines Tautomeren davon, jeweils in freier Form oder in Salzform, eine Verbindung der Formel

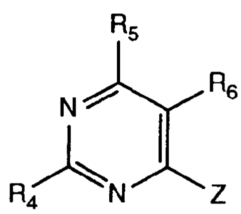

(II),

worin $R_4$, $R_5$ und $R_6$ die für die Formel I angegebenen Bedeutungen haben und Z ein leicht abspaltbarer nucleofuger Rest ist, oder gegebenenfalls ein Tautomeres davon mit einer Verbindung der Formel

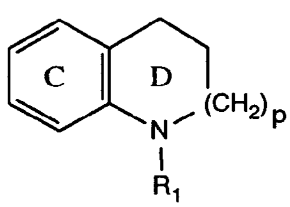

(III),

oder einem Salz davon, vorzugsweise in Gegenwart einer Base, umsetzt und worin $R_1$ und p die für die Formel I angegebenen Bedeutungen haben und mit einer einzigen Ausnahme der Ring C für den Ring A und der Ring D für den Ring B steht, wobei die Ringe A und B die für die Formel I angegebenen Bedeutungen haben, und wobei die erwähnte Ausnahme darin besteht, dass anstelle der in Formel I erwähnten Gruppe Ia die Gruppe der Formel $H(R_7')N-(C(R_2)(R_3))$ (IIIa) steht, worin $R_7$ Wasserstoff, $C_1$-$C_8$-Alkyl oder Benzyl ist und $R_2$, $R_3$, die für die Formel I angegebenen Bedeutungen haben, oder

b) zur Herstellung einer Verbindung der Formel I, worin $R_7$ die für die Formel I angegebene Bedeutung hat, jedoch von Wasserstoff, $C_1$-$C_8$-Alkyl und Benzyl verschieden ist, oder gegebenenfalls eines Tautomeren davon, jeweils in freier Form oder in Salzform, in eine Verbindung der Formel I, worin $R_7$ Wasserstoff ist, oder gegebenenfalls ein Tautomeres davon, jeweils in freier Form oder in Salzform, den gewünschten, von Wasserstoff, $C_1$-$C_8$-Alkyl und Benzyl verschiedenen, Substituenten $R_7$ durch N-Alkanoylierung, N-Benzoylierung, N-Alkylthiolierung, N-Phenylthiolierung oder N-Benzylthiolierung einführt

und jeweils, wenn erwünscht, eine verfahrensgemäss oder auf andere Weise erhältliche Verbindung

der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

**11.** Mittel zum Schutz von Pflanzen gegen den Befall durch Schädlinge, dadurch gekennzeichnet, dass es mindestens eine Verbindung gemäss Anspruch 1 oder gegebenenfalls ein Tautomeres davon, jeweils in freier Form oder in agrochemisch verwendbarer Salzform, als Wirkstoff und mindestens einen Hilfsstoff enthält.

**12.** Mittel gemäss Anspruch 11 zum Schutz von Pflanzen gegen den Befall durch phytopathogene Mikroorganismen und/oder Schädlinge der Ordnung Akarina und der Klasse Insecta.

**13.** Verfahren zur Herstellung eines Mittels gemäss Anspruch 11, dadurch gekennzeichnet, dass man den Wirkstoff mit dem (den) Hilfsstoff(en) innig vermischt.

**14.** Verwendung einer Verbindung gemäss Anspruch 1 oder gegebenenfalls eines Tautomeren davon, jeweils in freier Form oder in agrochemisch verwendbarer Salzform, oder eines Mittels gemäss Anspruch 11 oder 12 zum Schutz von Pflanzen gegen den Befall durch Schädlinge.

**15.** Verwendung gemäss Anspruch 14 zum Schutz von Pflanzen gegen den Befall durch phytopathogene Mikroorganismen und/oder Schädlinge der Ordnung Akarina und der Klasse Insecta.

**16.** Verfahren zum Schutz von Pflanzen gegen den Befall durch Schädlinge, dadurch gekennzeichnet, dass man als Wirkstoff eine Verbindung gemäss Anspruch 1 oder gegebenenfalls ein Tautomeres davon, jeweils in freier Form oder in agrochemisch verwendbarer Salzform, auf die Pflanzen, auf Teile der Pflanzen und/oder auf den Standort der Pflanzen appliziert.

**17.** Verfahren gemäss Anspruch 16 zum Schutz von Pflanzen gegen den Befall durch phytopathogene Mikroorganismen und/oder Schädlinge der Ordnung Akarina und der Klasse Insecta.

**18.** Eine Verbindung der Formel

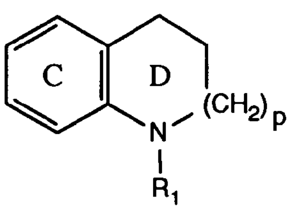

(III),

in freier Form oder in Salzform, worin $R_1$ und p die für die Formel I angegebenen Bedeutungen haben und mit einer einzigen Ausnahme der Ring C für den Ring A und der Ring D für den Ring B steht, wobei die Ringe A und B die für die Formel I angegebenen Bedeutungen haben, und wobei die erwähnte Ausnahme darin besteht, dass anstelle der in Formel I erwähnten Gruppe Ia die Gruppe der Formel $H(R_7')N-(C(R_2)(R_3))$ (IIIa) steht, worin $R_7$ Wasserstoff, $C_1$-$C_8$-Alkyl oder Benzyl ist und $R_2$, $R_3$, die für die Formel I angegebenen Bedeutungen haben, mit der Massgabe, dass wenn p 1 ist, die Gruppe IIIa in 2, 3, 6 oder 7 Position des aus den beiden Ringen C und D gebildeten bicyclischen Ringsystems gebunden ist, und mit der weiteren Massgabe, dass, wenn p 2 ist, die Gruppe IIIa in 7 oder 8 Position der aus den beiden Ringen C und D gebildeten bicyclischen Ringsystems gebunden ist.

Europäisches Patentamt

# EUROPÄISCHER RECHERCHENBERICHT

Nummer der Anmeldung

EP 93 81 0059
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 057 440 (SANKYO COMPANY LTD.)<br>* Ansprüche *<br>--- | 1,11 | C07D401/12<br>A01N43/54<br>C07D403/12 |
| X | CHEMICAL ABSTRACTS, vol. 115, 1991, Columbus, Ohio, US;<br>abstract no. 282072s,<br>U. ECKSTEIN 'Triarylmethane color formers, their preparation and use.'<br>* Zusammenfassung *<br>* RN 137780-88-2 *<br>& EP-A-0 433 813 (BAYER AG)<br>--- | 18 | |
| X | CHEMICAL ABSTRACTS, vol. 103, 1985, Columbus, Ohio, US;<br>abstract no. 510m,<br>J.R. HUFF ET AL. 'Bioactive conformation of 1-arylpiperazines at central serotonin receptors.'<br>* Zusammenfassung *<br>* RN 96430-18-1, RN 96430-17-0 *<br>& 'J. Med. Chem. 1985,28(7), 945-8'<br>--- | 18 | RECHERCHIERTE SACHGEBIETE (Int. Cl.5 ) |
| X | CHEMICAL ABSTRACTS, vol. 63, 1965, Columbus, Ohio, US;<br>abstract no. 4293a,<br>TETSUJI KAMETANI ET AL. 'Azabenzomorphan and related compounds. II. Synthesis of 1,2,3,4-tetrahydro-6H-1,5-methanobenzo(f)(1,4)diazocine.'<br>* Zusammenfassung *<br>* RN 94487-46-4 *<br>& 'Chem. Pharm. Bull. ( Tokyo ) 13(3), 295-9(1965)'<br>---<br>-/-- | 18 | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 11 MAI 1993 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung
EP 93 81 0059
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | CHEMICAL ABSTRACTS, vol. 113, 1990, Columbus, Ohio, US; abstract no. 78243c, R. JONES ET AL. 'Tetrahydrofolate coenzyme models: synthesis of tetrahydroimidazoisoquinolines and tetrahydroimidazoquinolines.' * Zusammenfassung * * RN 84393-46-4 * & 'J. Chem. Soc., Perkin Trans. 1 1990, (2), 385-91' --- | 18 | |
| X | CHEMICAL ABSTRACTS, vol. 89, 1978, Columbus, Ohio, US; abstract no. 163368c, V. F. VASIL'EVA ET AL. 'Substituted 1,2,3,4-tetrahydroquinolines. IV. 2-Aminomethyl derivatives' * Zusammenfassung * * RN 67868-46-4, RN 67868-34-2, RN 67868-32-0, RN 67868-31-9, RN 67868-30-8 * & 'Khim.-Farm Zh 1978, 12(5), 78-82' --- | 18 | RECHERCHIERTE SACHGEBIETE (Int. Cl.5 ) |
| X | CHEMICAL ABSTRACTS, vol. 81, 1974, Columbus, Ohio, US; abstract no. 120498n, H. C. RICHARDS '2-( Aminoalkyl)tetrahydroquinolines.' * Zusammenfassung * * RN 53425-78-8 * & US-A-3 821 228 (PFIZER INC.) --- -/-- | 18 | |

Der vorliegende Recherchenbericht wurde für alle Patentansprüche erstellt

| Recherchenort | Abschlußdatum der Recherche | Prüfer |
|---|---|---|
| DEN HAAG | 11 MAI 1993 | VAN BIJLEN H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 81 0059
Seite 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, vol. 76, 1972, Columbus, Ohio, US; abstract no. 41827n, C.A.R. BAXTER ET AL. 'Schistosomicides. 1. Derivatives of 2-aminomethyl-1,2,3,4-tetra hydroquinoline.' * Zusammenfassung * * RN 35521-72-3, RN 35521-64-3, RN 35517-26-1 * & 'J. Med. Chem. 1971, 14(11), 1033-42' --- | 18 | |
| X | CHEMICAL ABSTRACTS, vol. 71, 1969, Columbus, Ohio, US; abstract no. 30369k, H. C. RICHARDS '2-Aminoalkyltetrahydroquinolines as anthelmintics.' * Zusammenfassung * * RN 23099-07-2, RN 22989-40-8, RN 22989-39-5, RN 22982-89-4 * & SA-A-6 803 636 (PFIZER CORP.) --- | 18 | RECHERCHIERTE SACHGEBIETE (Int. Cl.5) |
| X | CHEMICAL ABSTRACTS, vol. 70, 1969, Columbus, Ohio, US; abstract no. 3950r, W.B. WRIGHT 'Central nervous system depressants. III. 2-Aminoalkyl-3,3a,4,5-te trahydroimidazo(1,5-a)quinolin-1(2H)-ones. ' * Zusammenfassung * * RN 21097-04-1, RN 21097-03-0, RN 21097-02-9 * & 'J. Med. Chem. 1968, 11(6),1161-4' --- -/-- | 18 | |

Der vorliegende Recherchenbericht wurde für alle Patentansprüche erstellt

| Recherchenort | Abschlußdatum der Recherche | Prüfer |
|---|---|---|
| DEN HAAG | 11 MAI 1993 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 81 0059
Seite 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, vol. 69, 1968, Columbus, Ohio, US; abstract no. 106505h, KOTLER-BRAJTBURG, J. 'Search for antiserotonin substances in the quinoline series. III. 3-(Aminomethyl) quinoline derivatives with a hydrogenated heterocyclic ring.' * Zusammenfassung * * RN 20146-17-2, RN 7521-71-3 * & 'Acta Pol. Pharm. 1968,25(2),117-21' --- | 18 | |
| X | CHEMICAL ABSTRACTS, vol. 65, 1966, Columbus, Ohio, US; abstract no. 15358a, TETSUJI KAMETANI ET AL. 'Studies on the synthesis of heterocyclic compounds. CXLI. Azabenzomorphan and related compounds. 9. A synthesis of 3-benzyl-3,4,5,6-tetrahydro -2H-1,5-methanobenzo(d)(1,3)diazocine.' * Zusammenfassung * * RN 7566-97-4 * & 'Chem. Pharm. Bull. 14(6), 566-71' --- | 18 | RECHERCHIERTE SACHGEBIETE (Int. Cl.5) |
| X | CHEMICAL ABSTRACTS, vol. 63, 1965, Columbus, Ohio, US; abstract no. 4292f, TETSUJI KAMETANI ET AL. 'Azabenzomorphan and related compounds. II. Synthesis of 1, 2,3,4-tetrahydro-6H-1,5-methanobenzo(f)(1, 4)diazocine.' * Zusammenfassung * * RN 1911-00-8 * & 'Chem. Pharm. Bull. 13(3), 295-9 (1965)' ----- | 18 | |

Der vorliegende Recherchenbericht wurde für alle Patentansprüche erstellt

| Recherchenort | Abschlußdatum der Recherche | Prüfer |
|---|---|---|
| DEN HAAG | 11 MAI 1993 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)